Europäisches Patentamt

European Patent Office

Office européen des brevets

(11) Publication number: **0 201 716 B1**

# EUROPEAN PATENT SPECIFICATION

(45) Date of publication of patent specification: **10.08.94**

(51) Int. Cl.⁵: **C12N  15/48**, C12N 1/20, C12P 21/02, A61K 39/21, G01N 33/569, C07K 15/00, //(C12N1/20,C12R1:19)

(21) Application number: **86104741.3**

(22) Date of filing: **07.04.86**

(54) **Expression of immunologically reactive viral proteins.**

(30) Priority: **08.04.85 US 721237**
**29.04.85 US 728052**
**10.07.85 US 753769**
**07.11.85 US 783299**

(43) Date of publication of application:
**20.11.86 Bulletin  86/47**

(45) Publication of the grant of the patent:
**10.08.94 Bulletin  94/32**

(84) Designated Contracting States:
**AT BE CH DE FR GB IT LI LU NL SE**

(56) References cited:
**EP-A- 0 173 529**
**EP-A- 0 181 150**
**EP-A- 0 185 444**
**EP-A- 0 187 041**

**SCIENCE, vol. 228, 5th April 1985, pages 93-96; N.T. CHANG et al.: "Expressionin Escherichia coli of open reading frame gene segments of HTLV-III"**

(73) Proprietor: **Bristol-Myers Squibb Company**
**345 Park Avenue**
**New York, N.Y. 10154(US)**

(72) Inventor: **Watanabe, Susan Mitsue**
**3031-14th Avenue West**
**Seattle Washington 98119(US)**
Inventor: **Cosand, Wesley**
**9022 Northeast 152nd Street**
**Bothell Washington 98011(US)**
Inventor: **McArdle, Susan**
**3911 Fourth Avenue**
**N.E., Apt D**
**Seattle Washington 98105(US)**
Inventor: **Travis, Bruce McFarland**
**6029 - 29th Avenue Northeast**
**Seattle Washington 98115(US)**

(74) Representative: **Patentanwälte Grünecker,**
**Kinkeldey, Stockmair & Partner**
**Maximilianstrasse 58**
**D-80538 München (DE)**

SCIENCE, vol. 227, 1st February 1985, pages 484-492; R. SANCHEZ-PESCADOR et al.: "Nucleotide sequence and expression of an AIDS-associated retrovirus (ARV-2)"

BIO/TECHNOLOGY, vol. 4, February 1986, pages 128-133; C.D. CABRADILLA et al.:"Serodiagnosis of antibodies to the human AIDS retrovirus with a bacteriallysynthesized ENV polypeptide"

EP 0 201 716 B1

**Description**

Technical Field

The present invention relates to an isolated DNA sequence comprising a portion of the *env* region of LAV genome according to claim 1, a recombinant plasmid capable of replication in bacterial host cells, a method for preparing a protein which is immunologically reactive with antibodies to LAV, a pharmaceutical composition and a protein comprising a portion of the LAV envelope protein.

Background Art

Acquired immune deficiency syndrome (AIDS) is a transmissible deficiency of cellular immunity characterized by opportunistic infections and certain rare malignancies. The dominant risk groups for AIDS include homosexually active males, intravenous drug abusers, recipients of transfusions and blood products, and the heterosexual partners and children of high-risk individuals, suggesting the involvement of an infectious agent transmitted through intimate contact or blood products.

Recent evidence indicates that the infectious agent responsible for disease transmission is a novel lymphotropic retrovirus, known as lymphadenopathy-associated virus (LAV) (Barré-Sinoussi et al., Science 220: 868 (1983)). Similar viruses have been reported by other scientific groups (Popovic et al., Science 224: 497 (1984); Levy et al., Science 225: 840 (1984)) and designated human T-cell lymphotropic virus type III (HTLV-III), AIDS-associated retrovirus (ARV), or immune deficiency-associated virus (IDAV). Still more recent data indicates that LAV, HTLV-III, ARV, and IDAV share several important characteristics, including substantial nucleotide homology (Wain-Hobson et al., Cell 40: 9 (1985); Muesing et al., Nature 313: 450 (1985); Sanchez-Pescador et al., Science 227: 484 (1985)), and should be considered isolates of the same virus, although there is a likelihood that strain-to-strain variations among the viral isolates will exist. In addition to exhibiting substantial nucleotide homology, the isolates are similar with respect to morphology, cytopathology, requirements for optimum reverse transcriptase activity, and at least some antigenic properties (Levy, supra; Schupbach et al., Science 224: 503 (1984)).

The virus causing the aquired immune deficiency syndrome (AIDS) has been the object of intensive research. As a result thereof, WO 86/02383 discloses a DNA sequence comprising a portion of the *env* region. Wain-Hobson et al., Cell 40: 9 (1985), mentioned above, disclose the complete nucleotide sequence of a molecular LAV clone designated lambda J19. Crowl et al., Cell 41: 979 (1985) disclose the nucleotide sequence of the envelope gene of a HTLV-III proviral genome including some restriction sites. Chang et al., Science 228, 93 (1985) discloses expression of *env* derived proteins in E. coli.

As noted above, the virus is known to be transmissable through blood products (blood, blood serum, blood plasma, and fractions thereof), making it important to screen the blood products to determine if the donor has been exposed to the virus. This can be done in any of several ways, including enzyme-linked immunosorbent assay (ELISA) for the detection of antibodies to LAV and related viruses. Individuals whose blood contains antibodies to LAV are said to be "seropositive." Blood from seropositive donors may be eliminated from the blood supply upon detection, thereby helping to prevent the spread of the disease.

The immune response of individuals exposed to LAV is variable. Antibodies can be produced to any of several viral proteins, including p13, p18, p25, p36, gp43, p55, gp110, etc. (Schupbach et al., New Engl. J. Med. 312: 265 (1985)). Not all individuals will make antibodies to the same proteins or to the same epitope on a given protein.

The detection of seropositive individuals, as currently practiced, has several inherent problems. Foremost among these problems is the need to isolate antigen from whole viruses for the immunological assays. This isolation requires the manipulation of large volumes of live, potentially infectious virus, and as such poses a significant safety hazard. In addition, there are concerns relating to the yield, purity, and reproducibility of virus from one preparation to another. This may result in an unacceptable number of false positives and/or negatives. Consequently, there is a need in the art for alternative methods of producing viral antigens which are useful in blood screening assays and which further provide other related advantages.

In addition, no effective treatment for the disease has yet been found. With the spread of AIDS beginning to reach extraordinary, if not epidemic proportions, and the extent to which the virus may be transmitted still in question, there is also a need in the art for a pharmaceutical composition to immunize the general population. The present invention fulfills this need and further provides other related advantages.

3

Disclosure of the Invention

Briefly stated, the present invention discloses DNA sequences comprising a portion of the envelope (env) region of the LAV genome, the portion coding for a protein which is immunologically reactive with antibodies to LAV. A recombinant plasmid capable of replication in bacterial host cells is also disclosed. The plasmid includes procaryotic transcriptional and translational signals for expression, followed in reading phase by the DNA sequence described above. In a preferred embodiment, signals are chosen from an operon, such as the trp operon, which is inducible and/or suppressible. Bacterial cells, such as E. coli, which have been transformed with the recombinant plasmid described above, are also disclosed.

Another aspect of the invention discloses a method for preparing proteins which are immunologically reactive with antibodies or T-cells reactive to LAV. The method comprises introducing into a bacterial host cell a recombinant plasmid capable of replication in bacterial host cells. The plasmid includes procaryotic transcriptional and translational signals for expression, followed in reading phase by a DNA sequence comprising a portion of the env region of the LAV genome, the portion coding for a protein which is immunologically reactive with antibodies to LAV. Subsequent to the introduction of the plasmid, the bacterial host is grown in an appropriate medium. Expression of the protein is then induced and the protein product of the sequence is isolated from the bacterial host. The protein product may be purified subsequent to isolation, as by gel-permeation chromatography.

A further aspect of the invention discloses a protein for use for determining the presence of antibodies to LAV in a biological fluid. The biological fluid is incubated with a protein produced by bacterial cells transformed with a recombinant plasmid as described above, thereby forming a reaction mixture, and subsequently the reaction mixture is analyzed to determine the presence of the antibodies. It is preferable that the step of analyzing the reaction mixture comprises contacting the reaction mixture with a labeled specific binding partner for the antibody. Alternatively, the presence of antibodies to LAV is determined by carrying out a competition between a sample suspected of containing antibody and labeled monoclonal antibody for binding to an immobilized recombinant protein. The reaction mixture is subsequently analyzed to determine the amount of labeled antibody bound to the solid phase.

Yet another aspect of the invention discloses a protein for use in an assay for determining the presence of LAV antigen in a biological fluid, said assay comprising incubating the biological fluid with a labeled protein produced by bacterial cells transformed with a recombinant plasmid as described above, and either sequentially or simultaneously, with an antibody to the protein such that specific binding occurs. Subsequently, the reaction mixture formed during the incubation may be analyzed to determine the amount of label associated with the antibody.

A protein for use in producing antibodies to LAV comprising immunizing an animal with a protein produced by bacterial cells transformed with a recombinant plasmid as described above, is also disclosed.

An additional aspect of the present invention discloses a protein for use in an assay for determining the presence of antibodies to LAV in a biological fluid, said assay comprising conjugating latex beads to a protein produced by bacterial cells transformed with a recombinant plasmid as described above. Subsequently, the biological fluid is incubated with the bead/protein conjugate, thereby forming a reaction mixture. The reaction mixture is then analyzed to determine the presence of the antibodies.

The proteins produced within the present invention, when used with a suitable carrier or diluent, form an immunologically effective vaccine composition. By administering to an individual an immunogenically effective amount of a protein encoded by a DNA sequence comprising a portion of the env region of the LAV genome attached to a physiologically acceptable carrier, infection caused by the virus responsible for AIDS can be prevented.

Other aspects of the invention will become evident upon reference to the following detailed description and attached drawings.

Brief Description of the Drawings

Figure 1 illustrates the construction of mpSW16 and mpSW19 from λJ19.

Figure 2 illustrates the trp E expression vectors pJH 11, pJH 12, and pJH 14, including the polylinker sequences.

Figure 3 illustrates the origin of the LAV inserts in pENV-1, pENV-2, pENV-3, pENV-4, and pENV-5.

Figure 4 illustrates the construction or pENV-5 and pENV-1 from pJH 12 and mpSW19.

Figure 5 illustrates the construction of pENV-2 from pJH 14 and mpSW19, and the construction of pENV-3 from pJH 11 and mpSW19.

Figure 6 illustrates the construction of pENV-4 from pJH 12 and mpSW16.

Figure 7 illustrates the amino acid sequence of ENV-3.
Figure 8 illustrates the nucleotide sequence of the env region and its protein product.

Best Mode for Carrying Out the Invention

Prior to setting forth the invention, it may be helpful to an understanding thereof to set forth definitions of certain terms to be used hereinafter.

Lymphadenopathy-Associated Virus (LAV): A human T-lymphotropic retrovirus. For purposes of the present invention, a virus is considered to be the same as or equivalent to LAV if it substantially fulfills the following criteria:

(a) the virus is tropic for T-lymphocytes especially T-helper cells (CD4[+], according to the international nomenclature defined in Bernard et al., eds., Leucocyte Typing, New York: Springer Verlag (1984));

(b) the virus is cytopathic for infected CD4[+] cells (rather than transforming, as are HTLV-I and II);

(c) the virus encodes an RNA-dependent DNA polymerase (reverse transcriptase) which is $Mg^{2+}$-dependent (optimum concentration 5 mM, optimum pH 7.8, not inhibitable by actinomycin D) and can employ oligo $(dT)_{12-18}$ as a primer for reverse transcription from its 3' LTR;

(d) the virus bands in a sucrose gradient at a density of approximately 1.16;

(e) the virus can be labeled with [$^3$H] uridine;

(f) the virus is distinct by immunological and nucleotide sequence criteria from members of the HTLV-I/II family of viruses (by this criterion HTLV-III is not to be considered a member of the HTLV-I/II family);

(g) the virus is substantially cross-reactive immunologically with the proteins encoded by the gag and env regions of LAV; and

(h) the virus shares substantial nucleotide homology (78-100%) and amino acid sequence homology (90-100%) with LAV.

Immunologically Reactive: An antigen and an antibody are said to be "immunologically reactive" if they are capable of binding specifically to each other, typically with an affinity of at least $10^6\,M^{-1}$, more often at least $10^8\,M^{-1}$.

Transformed or Transformation: The process of stably and heritably altering the genotype of a recipient cell or microorganism by the introduction of purified DNA.

Lymphadenopathy-associated virus (LAV) can be isolated from patients with AIDS or lymphadenopathy syndrome. The lymph nodes of such patients are typically biopsied and placed in culture medium supplemented as necessary to support growth. A mitogen such as interleukin-2 (IL-2) or phytohemagglutinin (PHA) can be included; antiserum to human interferon can also be included. Reverse transcriptase activity typically appears about day 15 of culture, indicating the presence of virus. The virus can be concentrated from the culture supernatant using a nonionic detergent, followed by banding in a sucrose gradient. These and other methods of purification are well known in the art and are described, for example, in Montelaro et al., J. Virology 42: 1029 (1982).

LAV can be propagated in any of a number of ways. It can be cultured in T-lymphocytes derived from umbilical cord or peripheral blood or bone marrow. Alternatively, it can be propagated in immortalized T-cells or B-cells; see, for example, Popovic et al., Science 224: 497 (1984), and Montagnier et al., Science 225: 63 (1984). Growth of the virus is usually monitored by the presence of reverse transcriptase activity.

A genomic clone of LAV can be prepared by any of several methods well known in the art, including but not limited to those described by Hahn et al., Nature 312: 166 (1984); Alizon et al., Nature 312: 757 (1984): Luciw et al., Nature 313: 760 (1984); and Muesing et al., Nature 313: 450 (1985).

Briefly, in one of these methods (Alizon et al.) DNA is isolated from LAV-infected T-cells of a healthy donor, partially digested with a restriction endonuclease such as Hind III, and the resultant digest fractionated electrophoretically. Fragments which correspond in size to the size of the entire LAV genome (approximately 9.2 Kb) are eluted from the gel, precipitated, resuspended, and ligated into the arms of an appropriately restricted vector. The ligation mix is packaged into bacteriophage particles. Bacteria are transfected with the bacteriophage, and the clones are screened in situ for LAV inserts using a suitable probe (such as a cDNA made from LAV-RNA). From such a clone, the desired region of LAV can be subcloned into a bacterial plasmid vector, such as pUC 18. Further subcloning can be desirable to remove unwanted sequences and to add additional restriction sites (in the form of a polylinker) at either end for the purpose of facilitating cloning into an expression vector.

The LAV sequences can then be subcloned into an inducible expression vector. A variety of expression vectors are known in the art and include λgt 11:Tn5 (Hall et al., Nature 311: 379 (1984); trp E (Paul et al., Euro. J. Cell Biol. 31: 171 (1983); pINIII (Masui et al., Biotechnology, Jan. 1984, p. 81).

The resultant proteins can be partially purified and used for a variety of purposes, including, as immunogens and antigens in immunoassays. For use as immunogens, the proteins can be injected into an animal, such as a mouse, rabbit, goat, etc., either in buffered solution or in adjuvant. Alternatively, the proteins can be purified by polyacrylamide gel electrophoresis and the bands of interest cut out from the gel, triturated, and resuspended in buffer for injection into the host aminal. Polyclonal or monoclonal antibodies can be prepared. For use as antigens in immunoassays, the proteins can be employed in labeled or unlabeled form. Where they are labeled, the labels can include radioisotopes, fluorophores, enzymes, luminescers, or particles. These and other labels are well known in the art and are described, for example, in the following U.S. patents: 3,766,162; 3,791,932; 3,817,837; 3,996,345; and 4,233,402.

Assays employing the recombinant proteins of the instant invention can be heterogenous (i.e., requiring a separation step) or homogeneous. If the assay is heterogeneous, a variety of separation means can be employed, including centrifugation, filtration, chromatography, or magnetism.

One preferred assay for the screening of blood products or other physiological fluids for the presence of antibodies is an ELISA assay. Typically, antigen (in this case, one or a combination of recombinant proteins) is adsorbed to the surface of a microtiter well. Residual protein-binding sites on the surface are then blocked with an appropriate agent, such as bovine serum albumin (BSA), heat-inactivated normal goat serum (NGS), or BLOTTO (a buffered solution of nonfat dry milk which also contains a preservative, salts, and an antifoaming agent). The well is then incubated with a sample suspected of containing specific antibody. The sample can be applied neat, or, more often, it can be diluted, usually in a buffered solution which contains a small amount (0.1-5.0% by weight) of protein, such as BSA, NGS, or BLOTTO. After incubating for a sufficient length of time to allow specific binding to occur, the well is washed to remove unbound protein and then incubated with a labeled, anti-human immunoglobulin antibody ($\alpha$HuIg). The label can be chosen from a variety of enzymes, including horseradish peroxidase (HRP), $\beta$-galactosidase, alkaline phosphatase, and glucose oxidase. Sufficient time is allowed for specific binding to occur, then the well is again washed to remove unbound conjugate, and the substrate for the enzyme is added. Color is allowed to develop and the optical density of the contents of the well is determined visually or instrumentally.

For convenience, reagents for ELISA assays may be provided in the form of kits. These kits can include microtiter plates to which viral proteins made by recombinant techniques have been pre-adsorbed, various diluents and buffers, labeled conjugates for the detection of specifically bound antibodies, and other signal-generating reagents, such as enzyme substrates, cofactors, and chromogens.

Due to the fact that a significant number of AIDS patients are highly immunosuppressed and have progressively lost the ability to make high-titer antibodies, the present invention utilizes portions of the envelope (env) region of the LAV genome which codes for a protein which is immunologically reactive with antibodies to LAV. The presence of antibodies to the envelope glycoprotein of LAV is a better indicator of LAV infection than is the presence of antibodies to other viral proteins, because the antibody titers to the envelope glycoprotein are thought to persist during the later stages of the disease, while antibody to other proteins, such as the core protein, may decline to less than detectable levels.

Another application of the recombinant proteins of this invention is use as human vaccines. The recombinant proteins can be extensively purified and formulated in a convenient manner, generally in concentrations of 1 ug to 20 mg per kg of host. Physiologically acceptable carriers, such as sterile water, saline, buffered saline, etc., can be employed. Adjuvants, such as aluminum hydroxide, can also be employed. The vaccine can be administered by intravenous, subcutaneous, intramuscular, or peritoneal injection. One injection can be sufficient, but more often, multiple injections at weekly to monthly intervals are preferred.

Alternatively, vaccinia virus recombinants can be constructed which express regions of the LAV genome. for example, the constructs of this invention can be inserted into a plasmid such as pMM34 (Mackett, et al., Science 227: 433, 1985) and vaccinia virus hybrids containing the resultant chimeric plasmid, formed by homologous recombination. Immunization with such recombinant virus vaccines has been shown to be effective in eliciting protective immunity in aminals to hepatitis B virus and vesicular stomatitis virus (Smith et al., Nature 311: 578, 1984).

The use of a recombinant protein vaccine in this manner eliminates the need to compose vaccines from inactivated preparations or avirulent strains of pathogenic microorganisms.

The genetic organization of LAV, 5'LTR-gag-pol-Q-env-F-3'-LTR, is unique among retroviruses. In all other known replication-competent retroviruses, the pol and env genes overlap; in LAV, they are separated by an open reading frame Q (Wain-Hobson et al., Cell 40: 9 (1985)).

The env open reading frame has a possible initiation site (methionine codon) at the eighth triplet and hence is expected to code for a protein of molecular weight 97 kd. This is consistent with the apparent molecular weight (110 kd) of the LAV glycoprotein on denaturing polyacrylamide gels. There are 32

6

potential N-glycosylation sites in the env gene (Asn-x-Ser/Thr). There are also three hydrophobic regions, characteristic of retroviral envelope proteins, corresponding to a signal peptide (encoded by nucleotides 5815-5850), a second region (7315-7350), and a transmembrane segment (7831-7896). The second region is preceded by an Arg/Lys-rich region which is thought to represent a proteolytic processing site (Wain-Hobson, supra).

As noted above, antibodies to the LAV glycoprotein (the env gene product) appear early in the course of virus infection and persist in the later stages of disease as well (Kitchen et al., Nature 312: 367 (1984)). Antibodies to the glycoprotein are, therefore, a particularly good indicator of prior exposure to LAV, making the glycoprotein, or portions thereof, particularly useful in blood screening assays.

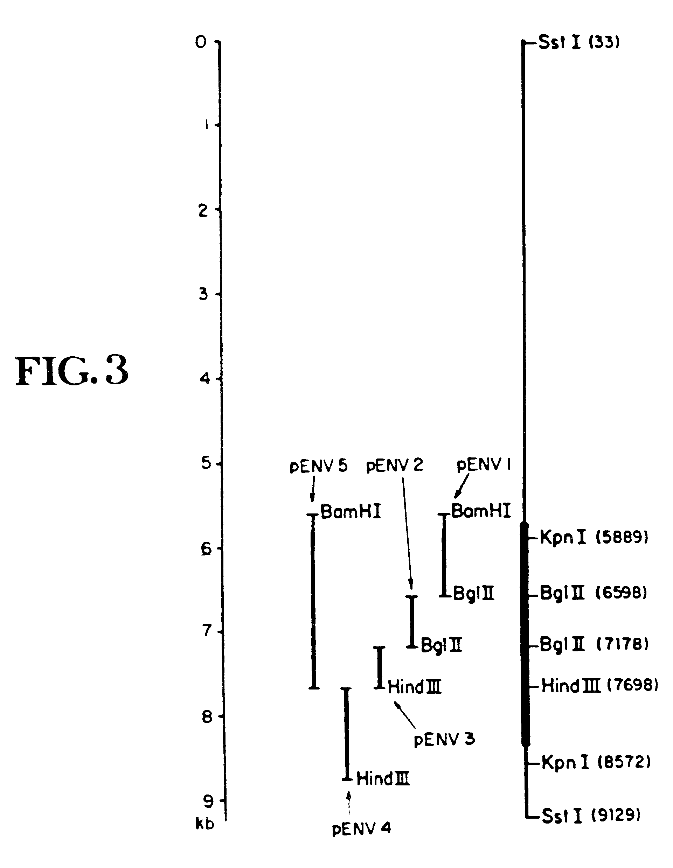

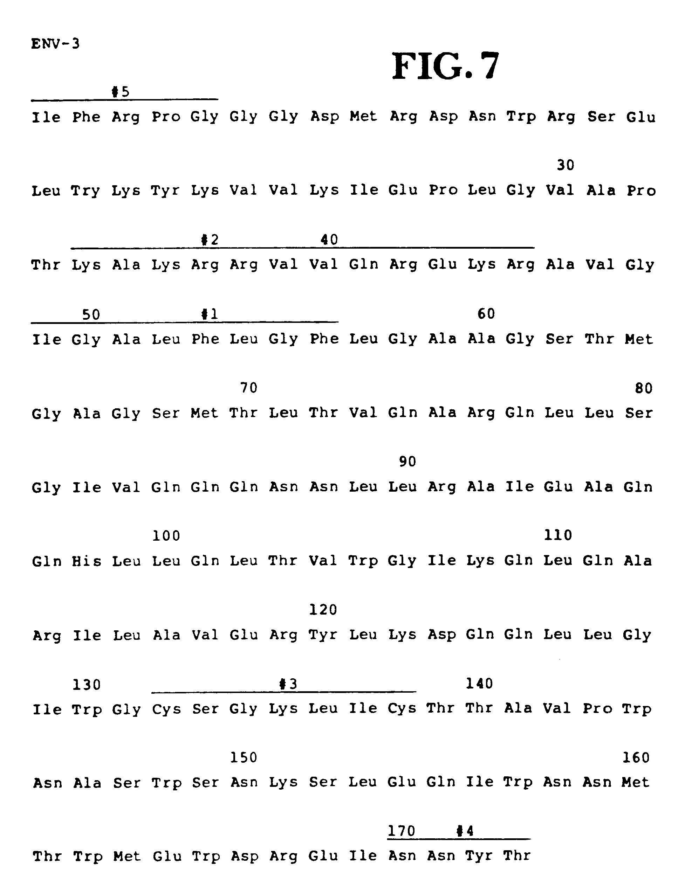

A particularly preferred portion of the env region region of the LAV genome is ENV-3. As shown in Figure 3, ENV-3 spans the nucleotide sequence from bp 7178 to bp 7698. Referring now to Figure 7, included within the region are the following sub-regions (overlined in Figure 7): (1) a hydrophobic peptide encoded by bp 7315 through 7350; (2) a dodecapeptide containing putative proteolytic processing sites for cleavage of the envelope precursor protein to gp 65 and gp 43; (3) a highly conserved region around two cysteine residues; (4) a conserved sequence at the 3' (carboxy) terminus; and (5) a conserved sequence at the 5' (amino) terminus.

Data obtained using synthetic peptides which correspond to the different sub-regions of ENV-3 indicate that the most important regions are those encompassing or in close proximity to the cysteine residues and those encompassing or in close proximity to the proteolytic processing region. The synthesis and screening of certain of these peptides are described in WO 86/06414, published November 6, 1986, the text of which is hereby incorporated by reference.

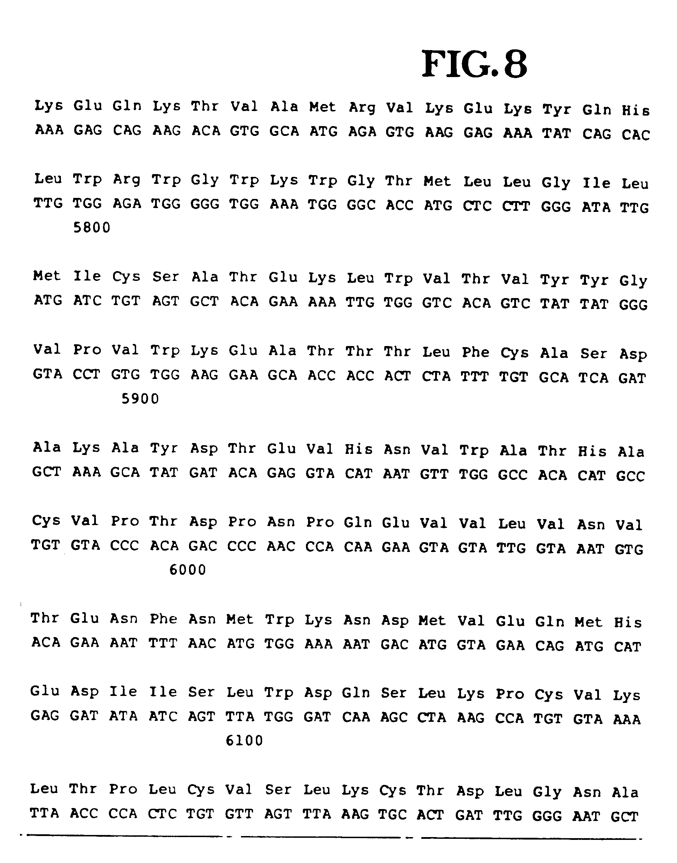

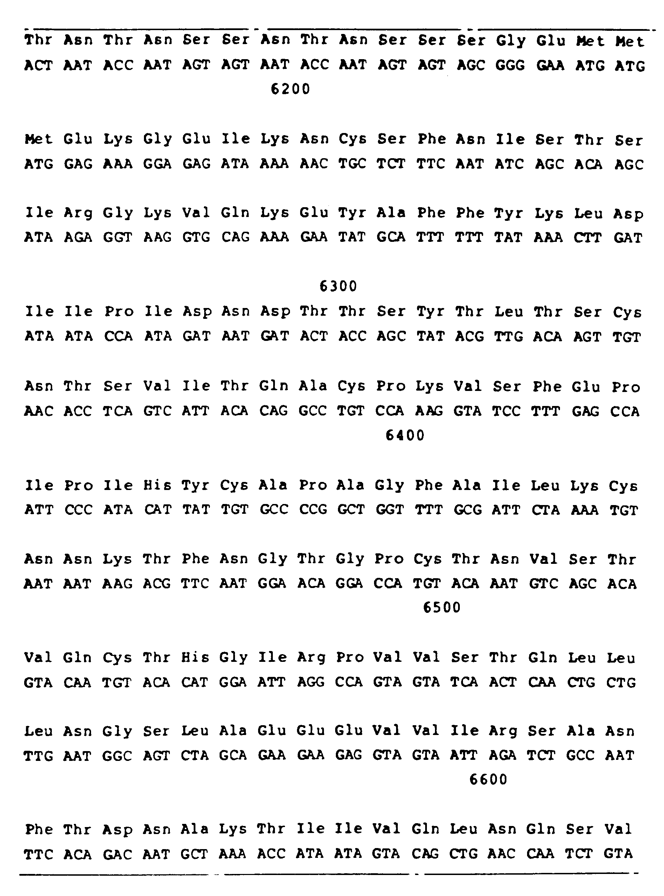

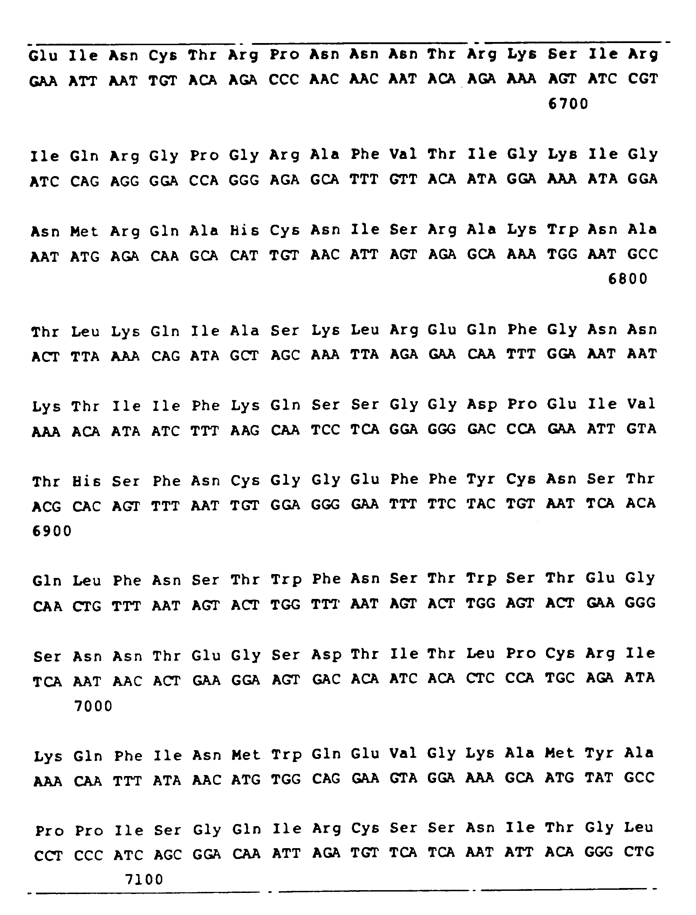

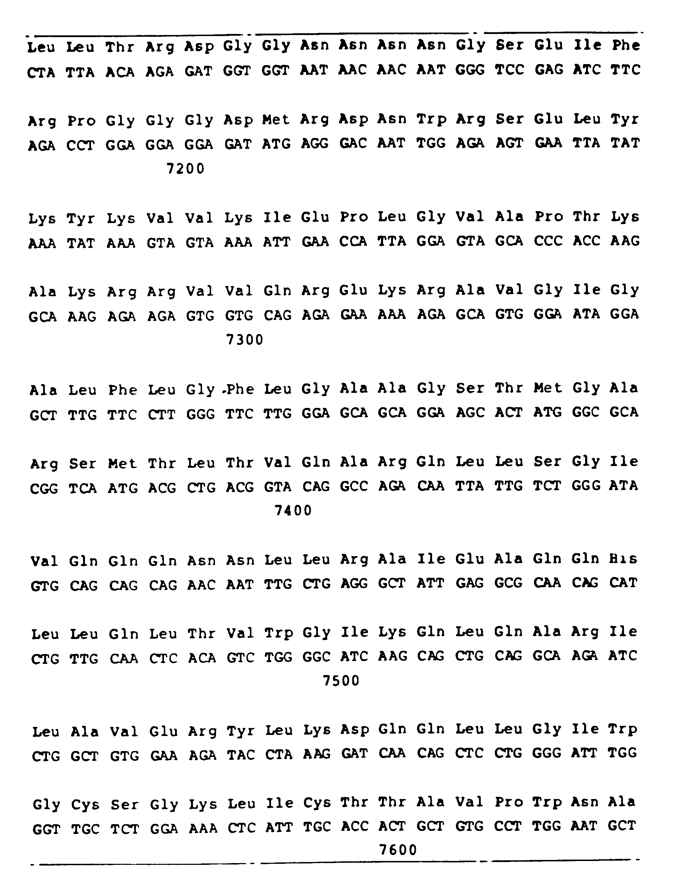

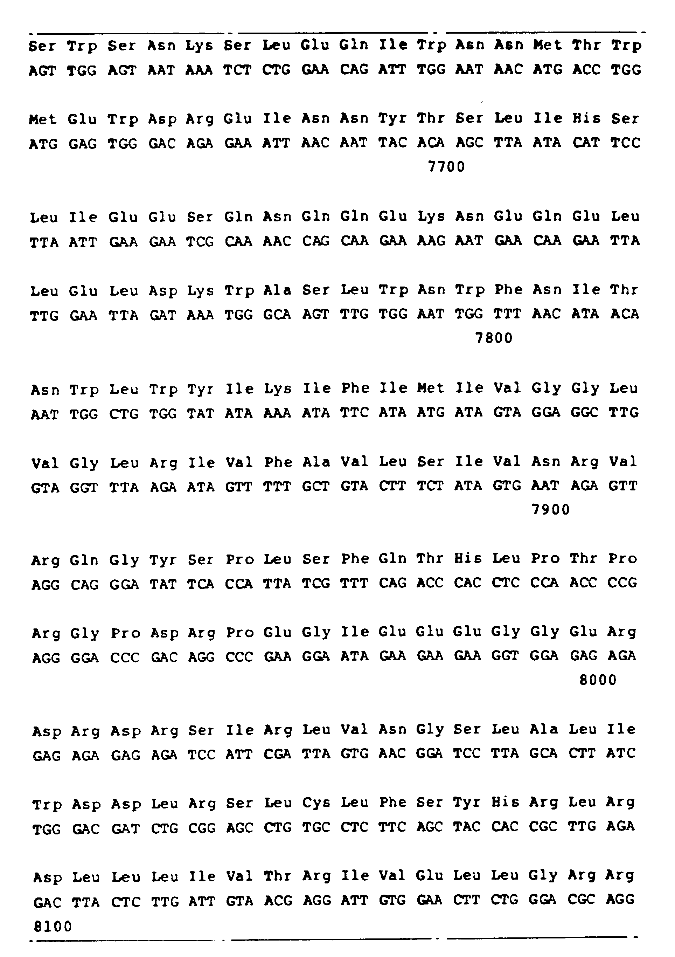

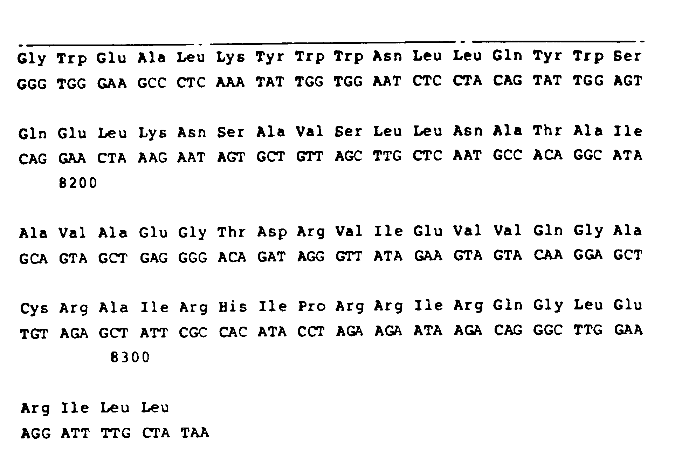

Additionally, ENV-3 is almost totally devoid of potential glycosylation sites; hence the antigenicity of the recombinant protein can be reasonably expected to mimic that of the native protein. The entire sequence of the env gene and its protein product is shown in Figure 8, in which the potential glycosylation sites are overlined. Referring to Figure 8, it can be seen that a large stretch of ENV-3 is devoid of such sites, and that in comparison to the remainder of the gene, ENV-3 as a whole is strikingly underglycosylated.

In the following example, a LAV genomic clone designated λJ19 was subcloned into the bacterial plasmid vector, pUC 18. The resultant subclone, designated pBT-1 (assigned ATCC Accession #53069), was further subcloned to yield pRS-3, which contained predominantly env, F, and LTR region sequences. The env and part of the F sequences were further subcloned into M13mp18, and then regions of the env sequence were transferred into the trp E inducible expression vector. The env DNA was inserted in-frame downstream of the trp E gene, resulting in the expression of a trp E-env fusion protein when E. coli were transformed with this construct. The resultant proteins were partially purified and characterized by their reactivity in ELISA with sera from known seropositive and known serongegative individuals. Five useful constructions, designated pENV-1 to pENV-5, were identified.

The following example is offered by way of illustration, and not by way of limitation.

EXAMPLE

## A. Construction of the trp-env expression vectors

Any of several bacterial expression systems can be used to express foreign proteins. The trp E system was chosen for the expression of LAV env sequences because it contains a strong inducible promotor, but its expression can also be suppressed so that foreign (and potentially toxic) protein does not accumulate within the bacteria for long period of time.

Since there is no rapid and convenient way to screen or select for bacteria transformed with vector containing a foreign insert (as opposed to bacterial transformed with vector alone), it is convenient to subclone the desired region of a large piece of DNA (such as λphage DNA) into a vector containing a selection system for inserts before transferring it to the trp E expression vector. This facilitates the screening of transformed bacteria for ones containing the desired insert.

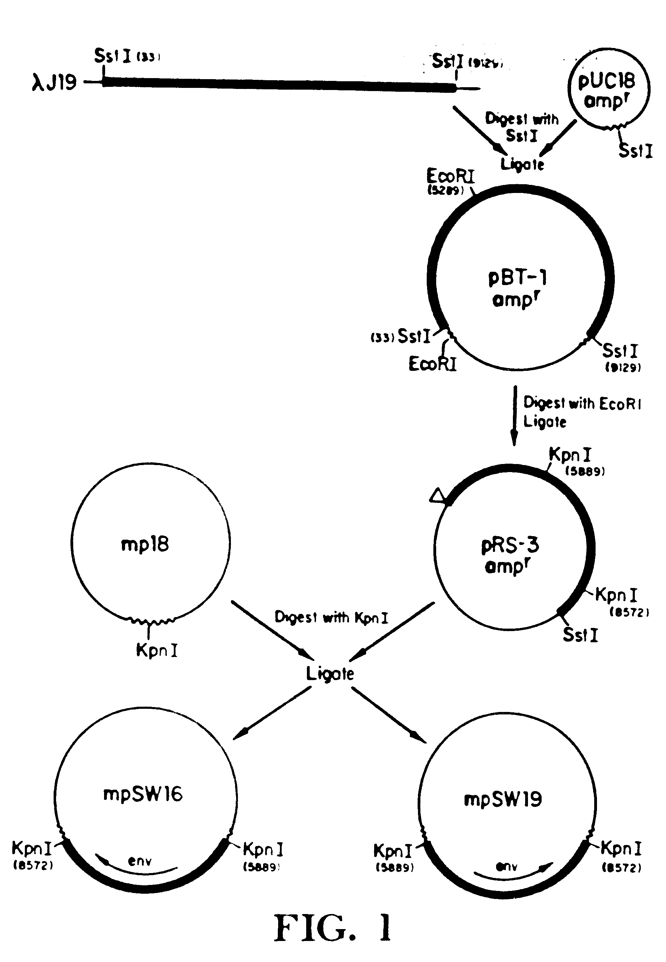

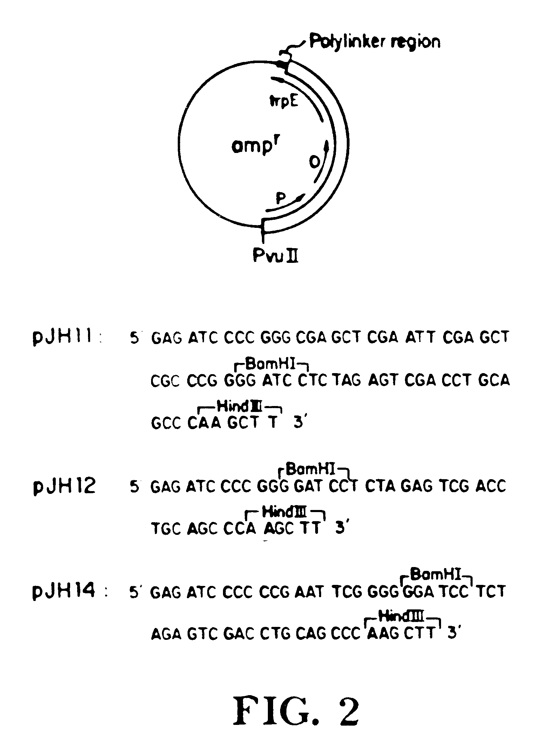

The approach of the inventors was, therefore, to first subclone in two steps most of the env region of the LAV genome into a transfer vector, M13mp18 (Figure 1). Then, various fragments of this subclone were ligated into either of three trp expression vectors (pJH 11, pJH 12, pJH 14) which differed only in the reading frame of the restriction sites in the polylinker region (see Figure 2). Some of the resulting subclones (e.g., pENV-5, pENV-4) were used as such, but others required further modifications (pENV-1, pENV-2, pENV-3) to produce the desired product.

## 1. Subcloning LAV genome

### a. Preparation of phage DNA

The entire LAV genome was obtained from the Pasteur Institut in the form of λphage particles containing a 9.2 Kb genomic DNA insert in the Hind III site of phage λL47.1. This clone is referred to as λJ19 and is described in Wain-Hobson et al., Cell 40: 9 (1985). λJ19 phage particles were transfected into the Q359 strain of E. coli K-12 (the genotype of Q359 is hsdRk⁻, hsdMk⁺, supF, φ80, P2) according to the procedure of Maniatis et al., Molecular Cloning: A Laboratory Manual, New York: Cold Spring Harbor Laboratory, 1982, at p. 64. A single plaque was picked and the phage amplified by the plate lysate method (Maniatis, supra, at p. 65). After a nine-hour incubation at 37°C, the plates (100 mm diameter) containing confluent plaques were overlaid with 5 ml of 100mM NaCl/20 mM MgSO₄/50 mM Tris, pH 7.5. After incubating for twelve hours at 4°C, the liquid was collected and extracted two times with an equal volume of chloroform.

To 10 ml of the resultant aqueous phase containing phage particles was added 2 ml 0.25 M EDTA/2.5% SDS/0.5 M Tris, pH 9, and the suspension was incubated at 70°C for fifteen minutes to disrupt the phage. 2.5 ml 8 M potassium acetate was added, and the solution was incubated on ice for fifteen minutes, then centrifuged for ten minutes at 12,000 xg at 4°C to pellet protein. The supernatant was transferred to a 50 ml polypropylene centrifuge tube and extracted with an equal volume of phenol (pH 8, equilibrated with 1 M Tris, pH 8) at 20°C. The aqueous phase was then extracted with an equal volume of chloroform:isoamyl-alcohol (24:1), at 20°C. To the aqueous phase was then added 2.5 volumes of 95% ethanol to precipitate the DNA. After centrifugation, the DNA pellet was dried and resuspended in 10 mM Tris HCl, pH 7.4/1 mM EDTA.

### b. Subcloning the env region

Approximately 12 ug of λJ19 DNA prepared in A.1.a. above was digested to completion with the restriction enzyme Sst I (Bethesda Research Labs, Bethesda, MD), which only cuts in the LTR regions of this isolate of LAV. The digest mixture was electrophoresed at 1 V/cm through 0.9% agarose in .089 M Tris-borate/.089 M boric acid/1 mM EDTA. The position of the 9.1 Kb fragment was determined relative to molecular weight standards after staining with ethidium bromide. This band was electroeluted into NA45 paper (Schleicher and Schuell, Keene, NH). The DNA was recovered from the paper according to instructions provided by the manufacturer.

The 9.1 kb Sst I fragment was ligated into the SST I digested vector pUC 18, at a ratio of 10 insert molecules: 1 vector molecule. E. coli strain HB101 was transformed with the ligation mix by the CaCl₂ procedure of Maniatis, et al. (supra) and plated onto LB plus ampicillin (200 ug/ml) agar plates.

Single colonies were picked and diluted into 3 ml LB plus ampicillin medium and grown overnight at 37°C with constant shaking. Plasmid DNA was prepared by the alkaline lysis method (Maniatis et al., supra, at p. 368). One colony was selected which contained the 9.1 Kb Sst I insert in an orientation such that the Eco RI site in the polylinker was closest to the 5' end of the LAV genome, as determined by restriction analysis of the plasmid DNA. This subclone was designated pBT-1 (ATCC Accession #53069) (Figure 1).

Plasmid pBT-1 was then digested with Eco RI, and the vector, still containing the 3' end of the LAV genome, was religated. This served to remove the 5' Eco RI fragments from the plasmid. HB101 cells were transformed with the ligation mixture, and a colony containing the insert pRS-3 was identified by restriction analysis of the purified plasmid DNA.

The sequence between the Kpn I sites at bp 5889 and bp 8572 [numbering according to Wain-Hobson et al., Cell 40: 9 (1985)], containing most of the env region, was then transferred from pRS-3 to the M13mp18 vector. This was done to further remove extraneous (non-env) sequences and also to place the env sequences in a vector which utilizes β-galactosidase rather than ampicillin as the selectable/screenable marker. This was advantageous because the trp E expression vector uses ampicillin resistance as its selectable marker; transfer of an insert from one vector (pUC 18) encoding the ampicillin resistance factor to another (trp expression vectors pJH 11, 12, 14) would make screening for the desired insert plus vector more difficult. M13 phage were screened by using the chromogenic substrate 5-bromo-4-chloro-3-indolyl-β-galactoside (Sigma Chemical Co.) to score for inactivation of β-galactosidase due to insertion of env sequence. Those phage containing inserts were screened for the orientation of the insert relative to restriction sites in the polylinker region. DNA was isolated from recombinants (mpSW16, mpSW19) for each orientation (Figure 1).

## 2. Insertion of the env sequence into trp vectors

The expression vectors contained the E. coli trp operon promotor, operator, and trp E gene inserted into pBR322 (Figure 2). The trp E gene was truncated at its 5'-most Bgl II site by the insertion of a polylinker sequence (Konopka et al., J. Virol 51: 223 (1984)). The different trp vectors (pJH 11, pJH 12, and pJH 14) differed according to the reading frame of the restriction sites within the polylinker region. Insertion of an open reading frame into the appropriate vector results in the production of a fusion protein with trp E sequences at the amino terminal end (Spindler et al., J. Virol 49: 132 (1984)).

The inventors selected five overlapping regions of LAV-env for expression (Figure 3). The choice was dictated by the limitation on insert size in these vectors (Konopka et al., J. Virol 51: 223 (1984)) and by the location of hydrophobic sequences, which may be deleterious for expression.

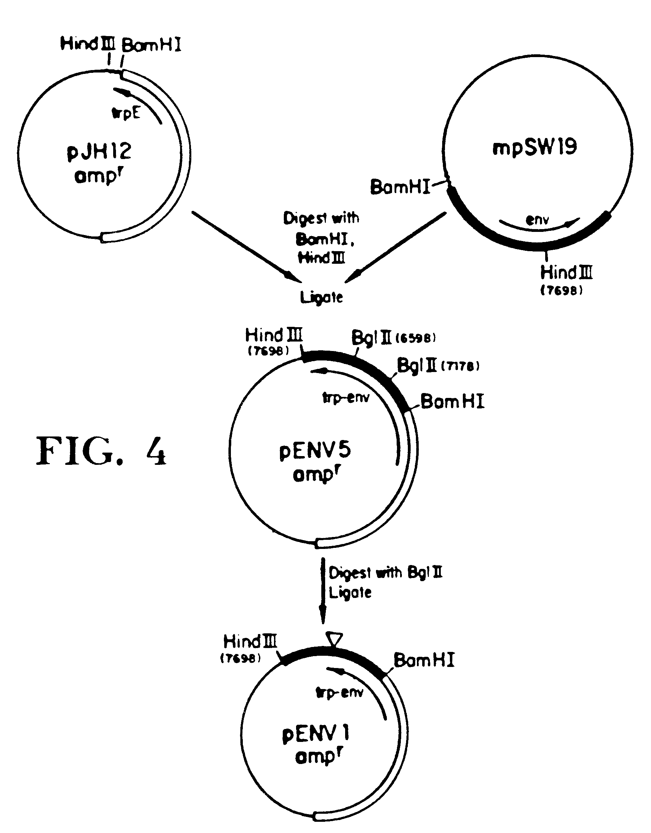

pENV-5 (ATCC Accession #53074) was constructed by ligating the env region from mpSW19's Bam HI site in the polylinker region to the env Hind III site (bp #7698) into Bam HI and Hind III (new England Biolabs) restricted pJH 12 (Figure 4). Ligations were transformed into CaCl$_2$-shocked E. coli HB101 and the bacterial plated in the presence of ampicillin (Sigma) at 100 ug/ml and tryptophan (Sigma) at 20 ug/ml. The tryptophan was added to suppress expression of the foreign protein, accumulation of which could be deleterious to the bacteria. Ampicillin-resistant colonies were screened by minilysates for the presence of recombinant plasmids containing the env fragment.

pENV-1 (ATCC Accession #53070) was derived from pENV-5 by deleting the sequences between the two Bgl II sites (bp 6598 and bp 7178 (Figure 4). This deletion decreased the size of the open reading frame by introducing a stop codon shortly after the Bgl II site.

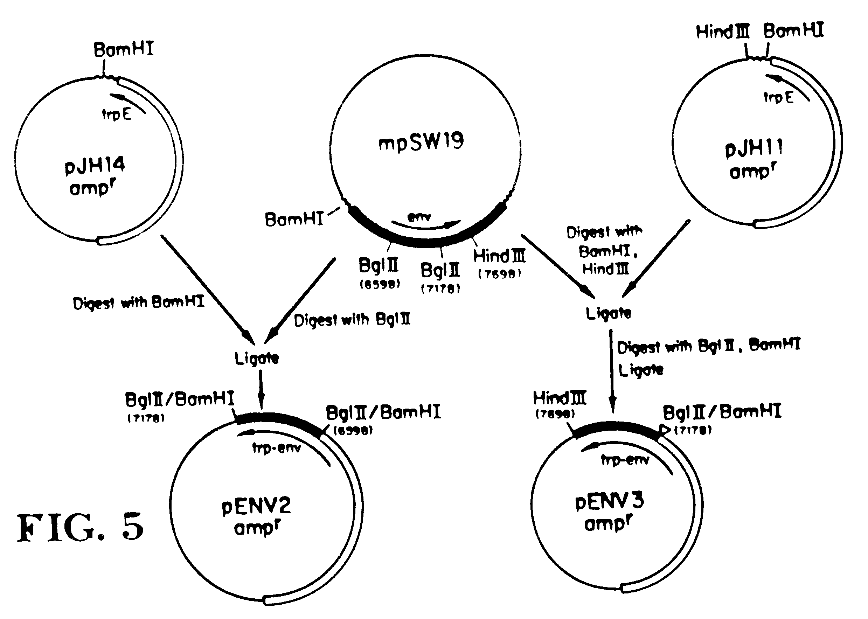

pENV-2 (ATCC Accession #53071) was constructed by inserting the fragment between two Bgl II sites (bp 6598 and bp 7178) into Bam HI restricted pJH 14 (Figure 5) (Bgl II and Bam HI overhangs are compatible). E. coli HB 101 were transformed with the ligation reactions and the ampicillin-resistant colonies screened by restriction analysis for the presence and orientation of the env insert into pJH 14.

pENV-3 (ATCC Accession #53072) required ligation of the env region betweeen mpSW19's Bam HI site (in the polylinker) and Hind III site (bp 7698) into Bam HI and Hind III restricted pJH 11 (Figure 5). E. coli HB101 were transformed with the ligation reaction, and the ampicillin-resistant colonies were screened by minilysates. DNA from an appropriate colony was isolated, digested with BAM HI and Bgl II (New England Biolabs), and religated. This DNA was used to transform E. coli HB101. The resultant ampicillin-resistant bacteria were screened by restriction analysis for recombinant plasmids which had deleted the Bam HI to Bgl II fragment (Figure 5). Deletion of the fragment brought the env sequences between the Bgl II site (bp 7178) and the Hind III site (bp 7698) into the correct reading frame.

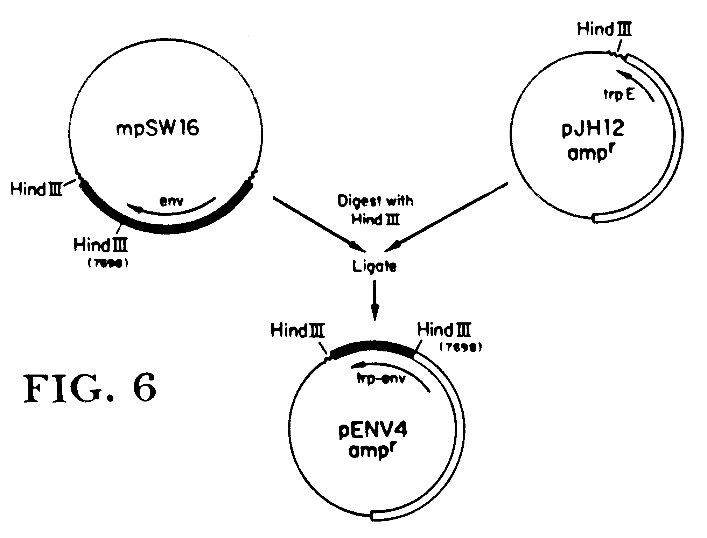

pENV-4 (ATCC Accession #53073), was constructed by ligating the Hind III fragment from mpSW16 into pJH 12 which has been treated with Hind III and calf intestinal alkaline phosphatase (Boehringer Mannheim) (Figure 6). Recombinant plasmids were identified by transforming E. coli HB101 with the ligation mixture and then screening by restriction analysis of minilysates.

B. Protein expression

## 1. Transformation of E. coli with the trp-env constructs

Each of the recombinant trp-env expression plasmids was transferred from E. coli HB101 into E. coli C600 because the latter is a potentially better host for protein production. Transfer involved transformation of CaCl$_2$-shocked C600 with supercoiled DNA from minilysates of HB101. Bacteria were plated in the presence of ampicillin and tryptophan as described (Konopka et al., J. Virol. 51: 223 (1984)). Drug-resistant colonies were screened by minilysates to confirm the presence of the appropriate plasmid.

## 2. Expression of trp-env proteins

Growth and induction of E. coli C600 transformed by the trp expression vectors were as described (Spindler et al., J. Virol 49: 132 (1984); Konopka et al., J. Virol. 51: 223 (1984)). Briefly, minimal medium containing tryptophan (20 ug/ml) and ampicillin (100 ug/ml) was inoculated with transformed bacteria from glycerol stocks. Cultures were grown with aeration at 37°C overnight. The overnight cultures were then inoculated at 1:100 into fresh minimal medium containing ampicillin (100 ug/ml) but no tryptophan. These cultures were grown with aeration for 2-3 hours (up to early log phase) at 37°C. The inducer, 3-β-indoleacrylic acid (Sigma) was added to a final concentration of 20 ug/ml from freshly made stocks at 20 mg/ml in 95% ethanol.

Induced cultures were grown at 37°C with aeration for 4 to 5 hours and then pelleted and, optionally, frozen. Protein yields from pENV-1, pENV-2, and pENV-3 were typically between 10-30 mg/liter, while the yields from pENV-4 and pENV-5 were typically less than 1 mg/liter.

## C. Isolation and purification of trp-env proteins

Fusion proteins were partially purified from cell pellets as described (Konopka et al., J. Virol. 51: 223 (1984)). Briefly, bacteria were resuspended in 100 ml of 50 mM Tris, pH 7.5/0.5 mM EDTA/150 mM NaCl (TNE) per liter of induced culture. Lysozyme (Sigma) was added to a final concentration of 1 mg/ml. After fifteen minutes at 0°C, NP40 was added to the mixture to a final concentration of 0.2% for ten minutes at 0°C. 1-2 mg of DNase (Sigma) was then added with 150 ml of DNase buffer (150 mM NaCl/12 mM $MgCl_2$). Reaction mixtures were incubated for one hour at 0°C with frequent stirring. Insoluble proteins were then pelleted by centrifugation for fifteen minutes at 8000 xg at 0°C. Pellets were washed two times in TNE and then analyzed for the presence of insoluble proteins by denaturintg polyacrylamide gel electrophoresis. Proteins were visualized by staining with Coomassie Brilliant Blue R.

The pellet containing the insoluble material from one liter of bacteria was solubilised in 6 mls of 6 M guanidinium chloride/0.1 M Tris HCl, pH 7.8. To this solution was added approximately 60 mg of solid dithiothreitol (DTT), and the reduction was allowed to proceed for thirty minutes at 37°C.

The solubilized reduced protein was subjected to gel-permeation chromatography on a 9.4 mm x 25 cm GF-250 Zorbax column (Du Pont, Wilmington, DE). A 100 ul aliquot of the protein solution was injected onto the column and eluted with 6 M guanidinium chloride at a flow rate of 0.5 ml/minute. The optical density at 280 nm was monitored, and fractions were collected at 30-second intervals. Fraction number 32, i.e., the fraction eluting between 7.75 and 8.00 mls, was used for the subsequent assays. Note: Inclusion of DTT will result in formation of insoluble metal sulfides when inferior grades of guanidinium chloride are used.

The trp-env fusion proteins have also been purified as follows;

Fusion proteins were partially purified from cell pellets as an insoluble pellet (described above). The pellet containing the insoluble material from four liters of bacteria was solubilized in 4 mls of 6 M guanidinium chloride/0.1 M Tris HCl, pH 7.8. To this solution was added approximately 100 mg of solid dithiothreitol (DTT), and the reduction was allowed to proceed for one hour at 37°C.

The solubilized, reduced protein was subjected to gel-permeation chromatography on a 2.6 x 87 cm column of Fractogel TSK HW-50(S) (MCB Manufacturing Chemists, Gibbs-town, NJ). The 4 mls of reduced protein were pumped onto the column and eluted with $2 \times 10^{-4}$ M DTT/$2 \times 10^{-3}$ ethylenediaminetetraacetic acid (EDTA)/6 M guanidinium chloride (American Research Products Co., South Euclid, OH). The column was run at 0.5 mls/minute, the optical density at 280 nm was monitored, and fractions were collected at ten-minute intervals. Fractions 34 and 35, the two fractions from the center of the UV absorbing peak, were used in subsequent assays.

## D. Immunological Reactivity of trp-env proteins

1. Analysis by Western blots

Aliquots from the insoluble protein preparations expressed by pENV-1, pENV-2, pENV-3, pENV-4, and pENV-5 were solubilized in 2% sodium dodecylsulphate/100 mM Tris, pH 6.8/20% glycerol/1.5 M β-mercaptoethanol and electrophoresed on denaturing polyacrylamide gels. Proteins were electrotransferred onto nitrocellulose (BA85, Schleicher and Schuell, Keene, NH) and the filters blocked with 5% bovine serum albumin (Sigma). Filters were then probed with E. coli-adsorbed human sera pooled from AIDS patients. The filters were developed with HRP-conjugated goat αHuIg. The pool was reactive with all five trp-env fusion proteins but not with trp E protein alone.

2. Analysis by ELISA

Insoluble protein preparations of pENV-1, pENV-2, pENV-3, pENV-5, and trp E were solubilized in 3 M guanidinium chloride. Insoluble material was removed by centrifugation at 15,000 xg for five minutes. Solubilized protein concentrations were determined by the method of Bradford (Analytical Biochemistry 72: 248 (1976)). The proteins were diluted in 0.05 M carbonate/bicarbonate buffer (pH 9.6) to a final concentration of 1-2 ug/ml. Fifty ul aliquots were loaded per microtiter well and incubated at 4°C overnight. Plates were then blocked with BLOTTO (5% [w/v] nonfat dry milk/0.01% thimerosol/0.01% antifoam A in 0.01 M sodium phosphate, pH 7.2/0.15 M sodium chloride) for one hour at 37°C. Pooled seropositive sera, sera from male homosexuals or LAS patients, and sera from healthy heterosexuals were diluted 1:100 with a 1:1 mixture of BLOTTO and PBS (0.01 M sodium phosphate, pH 7.3/0.15 M NaCl), and 50 ul of diluted serum was added per well for one hour at 37°C. The sera were removed, and the plates were washed three times in wash buffer (0.15 M NaCl/0.05% {w/v} Tween 20) before adding 100 ul of the goat anti-human IgG/horseradish peroxidase conjugate (diluted 1:10,000 in 50 mM NaCitrate/0.05% Tween 20/1% heat-inactivated normal goat serum; obtained from Antibodies, Inc., Davis, CA) for one hour at 37°C. The conjugate was removed and the plates washed three times with 0.15 M NaCl/0.05% (w/v) Tween 20. The ELISA assay was developed by adding 100 ul/well of substrate solution (10 mg o-phenylenediamine in 50 ml 0.05 M sodium citrate, pH 7.0) for thirty minutes at room temperature. Reactions were stopped with 100 ul/well of 3N $H_2SO_4$ and the optical density at 490 nm determined by an automated ELISA reader. Proteins produced by pENV-1, pENV-2, pENV-3, and pENV-5 were all found to be reactive with known seropositive sera.

Further screening of the pENV-1, pENV-3, and pENV-5 encoded protein was carried out using material from the guanidinium chloride-solubilized pellets described above (the pENV-1 encoded protein was further purified by gel-permeation chromatography as described above) against a panel of thirty-one human serum samples. The panel included eight sera from healthy heterosexuals (defined as negative in a whole virus ELISA), two serum pools from AIDS patients, and twenty-one sera from individuals diagnosed as LAS (lymphadenopathy syndrome). Sera from LAS individuals were confirmed as seropositive in a whole virus ELISA. Twelve were further confirmed by Western blot analysis. The results are shown in Table 1. These results demonstrate that sera from AIDS or LAS patients were more reactive with pENV-1, pENV-3, and pENV-5 encoded protein than were sera from normal individuals. This delineation indicates that the trp-env fusion proteins can be used to screen for the presence of sera reactive with the AIDS causative virus, LAV.

## TABLE 1

### COMPARISON OF pENV1, pENV3, AND pENV5 WITH A WHOLE VIRUS LYSATE IN AN ELISA ASSAY FOR THE DETECTION OF ANTIBODIES TO LAV

| Serum No. | Diagnosis | ELISA Whole Virus Lysate[1] | pENV1 | pENV3 | pENV5 | Confirmed as Seropositive[2] |
|---|---|---|---|---|---|---|
| 501 | Positive control pool | 1.109 | 1.744 | 0.744 | 1.685 | yes |
| Y-1 CDC | Positive control pool | 2.000 | 1.564 | 0.854 | 1.499 | n.d.[4] |
| 120 | LAS[3] and/or homosexual | 1.540 | 1.650 | 1.086 | 1.371 | yes |
| 121 | LAS and/or homosexual | 1.483 | 2.207 | 1.083 | 1.233 | yes |
| 122 | LAS and/or homosexual | 1.283 | 1.318 | 0.752 | 1.465 | yes |
| 124 | LAS and/or homosexual | 1.189 | 2.068 | 1.158 | 1.316 | yes |
| 125 | LAS and/or homosexual | 1.232 | 2.233 | 1.255 | 1.802 | yes |
| 126 | LAS and/or homosexual | 1.233 | 0.878 | 0.689 | 0.816 | yes |
| 127 | LAS and/or homosexual | 1.046 | 1.398 | 0.899 | 1.495 | yes |
| 128 | LAS and/or homosexual | 1.284 | 1.542 | 0.746 | 1.189 | yes |
| 129 | LAS and/or homosexual | 1.081 | 0.719 | 0.484 | 0.944 | yes |
| 130 | LAS and/or homosexual | 0.912 | 1.541 | 1.000 | 1.547 | yes |
| 131 | LAS and/or homosexual | 1.220 | 1.208 | 0.794 | 1.120 | yes |
| 132 | LAS and/or homosexual | 1.237 | 1.245 | 0.735 | 1.127 | yes |

| Serum No. | Diagnosis | ELISA Whole Virus Lysate[1] | pENV1 | pENV3 | pENV5 | Confirmed as Seropositive[2] |
|---|---|---|---|---|---|---|
| 133 | LAS and/or homosexual | 1.250 | 1.513 | 0.881 | 1.472 | yes |
| 134 | LAS and/or homosexual | 1.050 | 1.684 | 0.800 | 1.660 | yes |
| 135 | LAS and/or homosexual | 1.310 | 0.705 | 0.516 | 1.005 | yes |
| 138 | LAS and/or homosexual | 1.302 | 0.710 | 0.730 | 0.934 | yes |
| 153 | LAS and/or homosexual | 2.000 | 1.444 | 0.830 | 1.369 | yes |
| 154 | LAS and/or homosexual | 1.41 | 0.815 | 0.662 | 0.959 | yes |
| 155 | LAS and/or homosexual | 1.069 | 1.824 | 1.199 | 1.644 | yes |
| 157 | LAS and/or homosexual | 1.349 | 1.679 | 1.098 | 1.433 | yes |
| 666 | Unknown | 2.000 | 1.737 | 1.172 | 1.682 | yes |
| 633 | Healthy heterosexual | 0.222 | 0.089 | 0.113 | 0.135 | not sero-positive |
| 637 | Healthy heterosexual | 0.097 | 0.067 | 0.065 | 0.183 | not sero-positive |
| 639 | Healthy heterosexual | 0.123 | 0.073 | 0.045 | 0.186 | not sero-positive |
| 641 | Healthy heterosexual | 0.199 | 0.100 | 0.075 | 0.236 | not sero-positive |

|  |  | ELISA |  |  |  |  |
| Serum No. | Diagnosis | Whole Virus Lysate[1] | pENV1 | pENV3 | pENV5 | Confirmed as Seropositive[2] |
| --- | --- | --- | --- | --- | --- | --- |
| 667 | Healthy hetersexual | 0.095 | 0.208 | 0.113 | 0.450 | n.d. |
| 1890 | Healthy heterosexual | n.d. | 0.093 | 0.082 | 0.311 | n.d. |
| 1891 | Healthy heterosexual | n.d. | 0.164 | 0.086 | 0.411 | n.d. |
| 1892 | Healthy heterosexual | n.d. | 0.055 | 0.045 | 0.169 | n.d. |

---

[1]Virus lysate was prepared by precipitating the supernatant of infected cell cultures with 10% polyethylene glycol (PEG 6000), then banding twice to equilibrium in a 20-60% sucrose gradient. The viral band at density 1.16 was removed and viral particles further concentrated by ultracentrifugation, then lysed in RIPA buffer (described in Footnote 2) containing 0.5% sodium dodecyl sulfate, then coated on wells of microtiter test plates.

[2]Radiolabelled LAV antigens were disrupted in RIPA buffer (Gilead et al., Nature 264:263 (1976)) and then were reacted with human serum. The resultant immune complexes were separated by binding to a Staphlococcus aureus adsorbent (Kessler, J. Immunology 115:1617 (1975)) followed by multiple washings. Immuneprecipitated antigens were analyzed by SDS Polyacrylamide gel electrophoresis (Laemmli, Nature 227:680, (1970)) followed by fluorography. Presence of either a p25 or gp43 band was considered necessary and sufficient to confirm a sample as seropositive.

[3]LAS=lymphadenopathy syndrome.

[4]n.d.=not done.

3. Fluorescence slide test for detection of serum antibody to LAV

Soluble protein produced as described above is conjugated to latex beads, and the protein/bead preparation is ethanol fixed onto microscope slides. An aliquot of patient serum is incubated with the protein/beads on a slide. The slides are washed, and FITC-labeled anti-human immunoglobulin in Evans blue counterstain is added. The slides are washed, and mounting medium and coverslip applied to each.

Alternatively, the protein/bead preparation is placed in test tubes for incubation with patient serum. The tubes are centrifuged and washed, and FITC-labeled anti-human immunoglobulin in Evans blue counterstain is added. The tubes are centrifuged and the supernatant aspirated. An aliquot of the beads is placed on a microscope slide and ethanol fixed, and coverslips are mounted.

All slides are examined by fluorescence microscopy. If test serum is antibody positive, beads appear as fluorescent green spheres; if test serum is antibody negative, beads appear as red spheres.

From the foregoing, it will be appreciated that, although specific embodiments of the invention have been described herein for purposes of illustration, various modifications may be made without deviating from the spirit and scope of the invention. Accordingly, the invention is not to be limited except as by the appended claims.

**Claims**

**Claims for the following Contracting States : BE, CH, DE, FR, GB, IT, LI, LU, NL, SE**

1. An isolated DNA sequence comprising a portion of the env region of the LAV genome, said portion coding for a protein which is immunologically reactive with antibodies to LAV and wherein said sequence is selected from the insert env-1 of plasmid pENV-1, ATCC No. 53070, the insert env-2 of plasmid pENV-2, ATCC No. 53071, the insert env-3 of plasmid pENV-3, ATCC No. 53072, the insert env-4 of plasmid pENV-4, ATCC No. 53073, the insert env-5 of plasmid pENV-5, ATCC No. 53074 or a DNA sequence encoding a protein, which protein is at least 90% homologous to the protein encoded by one of the inserts env-1 to env-5, and wherein said DNA sequence is at least 90% homologous to the sequence encoding env-1 to env-5.

2. A recombinant plasmid capable of replication in bacterial host cells, said plasmid including procaryotic transcriptional and translational signals for expression, followed in reading phase by the DNA sequence according to claim 1.

3. The recombinant plasmid of claim 2 wherein said signals are derived from the trp operon.

4. A bacterial cell transformed with a recombinant plasmid according to any of the previous claims 2 or 3.

5. The transformed cell of claim 4 wherein said bacterial cell is E. coli.

6. A method for preparing a protein which is immunologically reactive with antibodies to LAV, comprising:
   introducing into a bacterial host cell the recombinant plasmid according to claim 2 or 3;
   growing said bacterial host in an appropriate medium;
   and
   isolating the protein.

7. The method of claim 6, including, after isolation of said protein, purifying said protein by gel permeation chromatography.

8. A pharmaceutical composition comprising a protein produced according to any of the claims 6 or 7, and a physiologically acceptable carrier and/or diluent.

9. The pharmaceutical composition of claim 8, including an adjuvant.

10. A pharmaceutical composition for use in stimulating an individual's immune system against infection caused by the virus responsible for acquired immune deficiency syndrome containing a protein produced according to any of the claims 6 or 7.

11. A pharmaceutical composition for use in stimulating an individual's immune system against infection caused by the virus responsible for acquired immune deficiency syndrome containing a protein encoded by the DNA sequence of claim 1.

12. A protein which comprises a portion of the LAV envelope protein comprising the amino acid sequence of figure 7, from isoleucine, No. 1 to threonine, No. 173, which protein is immunologically reactive with antibodies to LAV.

13. A protein produced according to any of the claims 6 or 7 for use in an assay for determining the presence of antibodies to LAV in a biological fluid.

**14.** A protein produced according to any of the claims 6 or 7 for use in an assay for determining the presence of LAV antigen in a biological fluid.

**15.** A protein produced according to any of the claims 6 or 7 for use in producing antibodies to LAV.

**16.** A protein produced according to any of the claims 6 or 7 for use as a medicament.

**17.** A protein as encoded by the DNA sequence of claim 1 for use in stimulating an individual's immune system against infection caused by the virus responsible for acquired immune deficiency syndrome.

**Claims for the following Contracting State : AT**

**1.** A process for preparing an isolated DNA sequence comprising a portion of the *env* region of the LAV genome, said portion coding for a protein which is immunologically reactive with antibodies to LAV, by isolating the insert *env*-1 of plasmid p*env*-1, ATCC no. 53070, the insert *env*-2 of plasmid p*env*-2, ATCC no. 53071, the insert *env*-3 of plasmid p*env*-3, ATCC no. 53072, the insert *env*-4 of plasmid p*env*-4, ATCC no. 53073, the insert *env*-5 of plasmid p*env*-5, ATCC no. 53074, or by isolating a DNA sequence encoding a protein, which protein is at least 90% homologous to the protein encoded by one of the inserts *env*-1 to *env*-5, and wherein said DNA sequence is at least 90% homologous to the sequence encoding *env*-1 to *env*-5 in a manner known per se.

**2.** A process for preparing a recombinant plasmid capable of replication in bacterial host cells, wherein prokaryotic transcriptional and translational signals for expression in combination with a DNA sequence obtained according to claim 1 in reading phase are inserted into a plasmid.

**3.** A process according to claim 2, wherein said signals are derived from the trp operon.

**4.** A method for producing a bacterial cell, wherein the bacterial cell is transformed with a recombinant plasmid obtained according to any of claims 2 or 3.

**5.** The method according to claim 4, wherein said bacterial cell is E. coli.

**6.** A method for preparing a protein which is immunologically reactive with antibodies to LAV, comprising:
- introducing into a bacterial host cell the recombinant plasmid according to claim 2 or 3;
- growing said bacterial host in an appropriate medium; and
- isolating the protein.

**7.** The method of claim 6, including, after isolation of said protein, purifying said protein by gel permeation chromatography.

**8.** A method for preparing a pharmaceutical composition wherein a protein produced according to any of claims 6 or 7 is combined with a physiologically acceptable carrier and/or diluent.

**9.** The method according to claim 8, wherein an adjuvant is added to the composition.

**10.** A method for preparing a pharmaceutical composition for use in stimulating individual's immune system against infection caused by the virus responsible for acquired immune deficiency syndrome, wherein a protein produced according to any of claims 6 or 7 is formulated.

**11.** A method for preparing a pharmaceutical composition for use in stimulating an individual's immune system against infection caused by the virus responsible for acquired immune deficiency syndrome, wherein a protein encoded by the DNA sequence obtained according to claim 1 is formulated.

**12.** A method for preparing a protein which comprises a portion of the LAV envelope protein, which protein is immunologically reactive with antibodies to LAV, by producing a protein having the amino acid sequence of Figure 7 from isoleucine, No. 1 to threonine, No. 173, in a manner known per se.

**13.** Use of a protein according to any of claims 6 or 7, in an assay for determining the presence of antibodies to LAV in a biological fluid.

**14.** Use of a protein produced according to any of claims 6 or 7 in an assay for determining the presence of LAV antigen in a biological fluid.

**15.** Use of a protein produced according to any of claims 6 or 7 for producing antibodies to LAV.

**16.** Use of a protein according any of claims 6 or 7 for the preparation of a medicament.

**17.** Use of a protein as encoded by the DNA sequence provided according to claim 1 for preparing a medicament for stimulating an individual's immune system against infection caused by the virus responsible for acquired immune deficiency syndrome.

**Patentansprüche**
**Patentansprüche für folgende Vertragsstaaten : BE, CH, DE, FR, GB, IT, LI, LU, NL, SE**

**1.** Eine isolierte DNA-Sequenz, umfassend einen Teil der env-Region des LAV-Genoms, wobei der Teil für ein Protein kodiert, das immunologisch reaktionsfähig ist mit Antikörpern gegen LAV, und worin die Sequenz ausgewählt wird aus dem Insert env-1 von Plasmid pENV-1, ATCC Nr. 53070, dem Insert env-2 von Plasmid pENV-2, ATCC Nr. 53071, dem Insert env-3 von Plasmid pENV-3, ATCC Nr. 53072, dem Insert env-4 von Plasmid pENV-4, ATCC Nr. 53073, dem Insert env-5 von Plasmid pENV-5, ATCC Nr. 53074 oder einer DNA-Sequenz, die für ein Protein kodiert, wobei das Protein zu mindestens 90 % homolog zu dem Protein, kodiert von einem der Inserts env-1 bis env-5, ist, und worin die DNA-Sequenz zu mindestens 90 % homolog zu der Sequenz ist, die env-1 bis env-5 kodiert.

**2.** Ein rekombinantes Plasmid, das zur Replikation in bakteriellen Wirtszellen fähig ist, wobei das Plasmid procaryontische Transkriptions- und Translationssignale zur Expression enthält, gefolgt von der DNA-Sequenz gemäß Anspruch 1 im Leserastar.

**3.** Das rekombinante Plasmid nach Anspruch 2, worin die Signale von dem trp-Operon stammen.

**4.** Eine Bakterienzelle transformiert mit einem rekombinanten Plasmid nach einem der vorherigen Ansprüche 2 oder 3.

**5.** Die transformierte Zelle nach Anspruch 4, worin die Bakterienzelle E. coli darstellt.

**6.** Ein Verfahren zum Herstellen eines Proteins, das mit Antikörpern gegen LAV immunologisch reaktionsfähig ist, umfassend:
   - Einführen des rekombinanten Plasmids gemäß Anspruch 2 oder 3 in eine bakterielle Wirtszelle;
   - Kultivieren des bakteriellen Wirts in einem geeigneten Medium; und
   - Isolieren des Proteins.

**7.** Das Verfahren nach Anspruch 6, das nach der Isolierung des Proteins das Reinigen des Proteins durch Gelausschlußchromatographie umfaßt.

**8.** Eine pharmazeutische Zusammensetzung, enthaltend ein Protein, hergestellt gemäß einem der Ansprüche 6 oder 7, und einen physiologisch verträglichen Träger und/oder ein Verdünnungsmittel.

**9.** Die pharmazeutische Zusammensetzung nach Anspruch 8, die ein Adjuvanz umfaßt.

**10.** Eine pharmazeutische Zusammensetzung zur Verwendung bei der Stimulierung des Immunsystems eines Individuums gegen eine Infektion, die durch das Virus, das für das Syndrom der erworbenen Immunschwäche verantwortlich ist, verursacht ist, enthaltend ein Protein hergestellt nach einem der Ansprüche 6 oder 7.

**11.** Eine pharmazeutische Zusammensetzung zur Verwendung bei der Stimulierung des Immunsystems eines Individuums gegen eine Infektion, die durch das Virus, das für das Syndrom der erworbenen

EP 0 201 716 B1

Immunschwäche verantwortlich ist, verursacht ist, enthaltend ein Protein kodiert durch die DNA-Sequenz nach Anspruch 1.

**12.** Ein Protein, das einen Teil des LAV-Hüllproteins umfaßt, umfassend die Aminosäuresequenz von Figur 7 von Isoleucin, Nr. 1 bis Threonin, Nr. 173, wobei das Protein mit Antikörpern gegen LAV immunologisch reaktionsfähig ist.

**13.** Ein nach einem der Ansprüche 6 oder 7 hergestelltes Protein zur Verwendung in einem Assay zur Bestimmung der Anwesenheit von Antikörpern gegen LAV in einer biologischen Flüssigkeit.

**14.** Ein nach einem der Ansprüche 6 oder 7 hergestelltes Protein zur Verwendung in einem Assay zur Bestimmung der Anwesenheit von LAV-Antigen in einer biologischen Flüssigkeit.

**15.** Ein nach einem der Ansprüche 6 oder 7 hergestelltes Protein zur Verwendung bei der Herstellung von Antikörpern gegen LAV.

**16.** Ein nach einem der Ansprüche 6 oder 7 hergestelltes Protein zur Verwendung als Arzneimittel.

**17.** Ein Protein, wie durch die DNA-Sequenz nach Anspruch 1 kodiert, zur Verwendung bei der Stimulierung des Immunsystems eines Individuums gegen eine Infektion, die durch das Virus, das für das Syndrom der erworbenen Immunschwäche verantwortlich ist, verursacht ist.

**Patentansprüche für folgenden Vertragsstaat : AT**

**1.** Verfahren zum Herstellen einer isolierten DNA-Sequenz, umfassend einen Teil der env-Region des LAV-Genoms, wobei der Teil für ein Protein kodiert, das immunologisch reaktionsfähig ist mit Antikörpern gegen LAV, durch Isolieren des Inserts env-1 von Plasmid pENV-1, ATCC Nr. 53070, des Inserts env-2 von Plasmid pENV-2, ATCC Nr. 53071, des Inserts env-3 von Plasmid pENV-3, ATCC Nr. 53072, des Inserts env-4 von Plasmid pENV-4, ATCC Nr. 53073, des Inserts env-5 von Plasmid pENV-5, ATCC Nr. 53074 oder durch Isolieren einer DNA-Sequenz, die für ein Protein kodiert, wobei das Protein zu mindestens 90 % homolog zu dem Protein, kodiert von einem der Inserts env-1 bis env-5, ist, und worin die DNA-Sequenz zu mindestens 90 % homolog zu der Sequenz ist, die env-1 bis env-5 kodiert, in an sich bekannter Weise.

**2.** Verfahren zum Herstellen eines rekombinanten Plasmids, das zur Replikation in bakteriellen Wirtszellen fähig ist, worin procaryontische Transkriptions- und Translationssignale zur Expression in Kombination mit einer DNA-Sequenz, erhalten gemäß Anspruch 1, im Leseraster in ein Plasmid eingefügt werden.

**3.** Das Verfahren nach Anspruch 2, worin die Signale von dem trp-operon stammen.

**4.** Verfahren zum Herstellen einer Bakterienzelle, worin die Bakterienzelle mit einem rekombinanten Plasmid transformiert wird, das nach einem der Ansprüche 2 oder 3 erhalten wird.

**5.** Das Verfahren nach Anspruch 4, worin die Bakterienzelle E. coli darstellt.

**6.** Verfahren zum Herstellen eines Proteins, das mit Antikörpern gegen LAV immunologisch reaktionsfähig ist, umfassend:
   - Einführen des rekombinanten Plasmids gemäß Anspruch 2 oder 3 in eine bakterielle Wirtszelle;
   - Kultivieren des bakteriellen Wirts in einem geeigneten Medium; und
   - Isolieren des Proteins.

**7.** Das Verfahren nach Anspruch 6, das nach der Isolierung des Proteins das Reinigen des Proteins durch Gelausschlußchromatographie umfaßt.

**8.** Verfahren zum Herstellen einer pharmazeutischen Zusammensetzung, worin ein Protein, hergestellt nach einem der Ansprüche 6 oder 7, mit einem physiologisch verträglichen Trägermaterial und/oder einem Verdünnungsmittel kombiniert wird.

18

EP 0 201 716 B1

9. Das Verfahren nach Anspruch 8, worin ein Adjuvanz der Zusammensetzung zugesetzt wird.

10. Verfahren zum Herstellen einer pharmazeutischen Zusammensetzung zur Verwendung bei der Stimulierung des Immunsystems eines Individuums gegen eine Infektion, die durch das Virus, das für das Syndrom der erworbenen Immunschwäche verantwortlich ist, verursacht ist, worin ein Protein, hergestellt nach einem der Ansprüche 6 oder 7, formuliert wird.

11. Verfahren zum Herstellen einer pharmazeutischen Zusammensetzung zur Verwendung bei der Stimulierung des Immunsystems eines Individuums gegen eine Infektion, die durch das Virus, das für das Syndrom der erworbenen Immunschwäche verantwortlich ist, verursacht ist, worin ein Protein, das von der DNA-Sequenz, erhalten nach Anspruch 1, kodiert wird, formuliert wird.

12. Verfahren zum Herstellen eines Proteins, das einen Teil des LAV-Hüllproteins umfaßt, wobei das Protein immunologisch reaktionsfähig ist mit Antikörpern gegen LAV, indem ein Protein mit der Aminosäuresequenz aus Figur 7 von Isoleucin, Nr. 1 bis Threonin, Nr. 173 in an sich bekannter Weise hergestellt wird.

13. Verwendung eines Proteins nach einem der Ansprüche 6 oder 7 in einem Assay zur Bestimmung der Anwesenheit von Antikörpern gegen LAV in einer biologischen Flüssigkeit.

14. Verwendung eines Proteins, hergestellt nach einem der Ansprüche 6 oder 7, in einem Assay zur Bestimmung der Anwesenheit von LAV-Antigen in einer biologischen Flüssigkeit.

15. Verwendung eines Proteins, hergestellt nach einem der Ansprüche 6 oder 7, zur Herstellung von Antikörpern gegen LAV.

16. Verwendung eines Proteins nach einem der Ansprüche 6 oder 7 zur Herstellung eines Medikaments.

17. Verwendung eines Proteins, wie von der gemäß Anspruch 1 bereitgestellten DNA-Sequenz kodiert, zur Herstellung eines Medikaments zur Stimulierung des Immunsystems eines Individuums gegen eine Infektion, die durch das Virus, das für das Syndrom der erworbenen Immunschwäche verantwortlich ist, verursacht ist.

**Revendications**

**Revendications pour les Etats contractants suivants : BE, CH, DE, FR, GB, IT, LI, LU, NL, SE**

1. Séquence d'ADN isolée comprenant une portion de la région env du génome de LAV, ladite portion codant pour une protéine qui a une activité immunologique avec des anticorps à LAV et où ladite séquence est choisie parmi l'insert env-1 du plasmide pENV-1, ATCC No. 53070, l'insert env-2 du plasmide pENV-2, ATCC No. 53071, l'insert env-3 du plasmide pENV-3, ATCC No. 53072, l'insert env-4 du plasmide pENV-4, ATCC No. 53073, l'insert env-5 du plasmide pENV-5, ATCC No. 53074 ou bien une séquence d'ADN codant une protéine, laquelle protéine est homoloque au moins à 90% avec la protéine codée par l'un des inserts env-1 à env-5, et où ladite séquence d'ADN est homologue au moins à 90% avec la séquence codant env-1 à env-5.

2. Plasmide recombinant capable d'une réplication chez des cellules hôtes bactériennes, ledit plasmide comprenant des signaux procaryotes de transcription et de traduction pour l'expression avec ensuite une phase de lecture par la séquence d'ADN selon la revendication 1.

3. Plasmide recombinant de la revendication 2 où lesdits signaux sont dérivés de l'opéron trp.

4. Cellule bactérienne transformée par un plasmide recombinant selon l'une quelconque des revendications 2 ou 3 qui précèdent.

5. Cellule transformée de la revendication 4, où ladite cellule bactérienne est E. coli.

6. Méthode de préparation d'une protéine qui a une activité immunologique avec les anticorps à LAV comprenant :

19

l'introduction, dans une cellule hôte bactérienne, du plasmide recombinant selon la revendication 2 ou 3,

la croissance dudit hôte bactérien dans un milieu approprié, et

l'isolement de la protéine.

7. Méthode de la revendication 6, comprenant, après isolement de ladite protéine, la purification de ladite protéine par chromatographie par perméation de gel.

8. Composition pharmaceutique comprenant une protéine produite selon l'une quelconque des revendications 6 ou 7 et son support et/ou diluant acceptable en pharmacie.

9. Composition pharmaceutique de la revendication 8, comprenant un adjuvant.

10. Composition pharmaceutique à utiliser pour stimuler le système immunitaire d'un individu contre une contamination provoquée par le virus responsable du syndrome immunodéficitaire acquis, contenant une protéine produite selon l'une quelconque des revendications 6 ou 7.

11. Composition pharmaceutique à utiliser pour la stimulation du système immunitaire d'un individu contre une contamination provoquée par le virus responsable du syndrome immunodéficitaire acquis contenant une protéine codée par la séquence d'ADN de la revendication 1.

12. Protéine qui comprend une portion de la protéine d'enveloppe de LAV comprenant la séquence d'acides aminés de la figure 7, à partir de l'isoleucine, No. 1 à la thréonine, No. 173, laquelle protéine a une activité immunologique avec les anticorps à LAV.

13. Protéine produite selon l'une quelconque des revendications 6 ou 7, à utiliser dans un essai pour déterminer la présence d'anticorps à LAV dans un fluide biologique.

14. Protéine produite selon l'une quelconque des revendications 6 ou 7, à utiliser dans un essai pour déterminer la présence d'un antigène de LAV dans un fluide biologique.

15. Protéine produite selon l'une quelconque des revendications 6 ou 7, à utiliser dans la production d'anticorps à LAV.

16. Protéine produite selon l'une quelconque des revendications 6 ou 7, à utiliser comme médicament.

17. Protéine codée par la séquence d'ADN de la revendication 1, à utiliser pour stimuler un système immunitaire d'un individu contre une contamination provoquée par le virus responsable du syndrome immunodéficitaire acquis.

**Revendications pour l'Etat contractant suivant : AT**

1. Procédé de préparation d'une séquence d'ADN isolée comprenant une portion de la région env du génome de LAV, ladite portion codant pour une protéine qui a une activité immunologique avec des anticorps à LAV, en isolant l'insert env-1 du plasmide pENV-1, ATCC No. 53070, l'insert env-2 du plasmide pENV-2, ATCC No. 53071, l'insert env-3 du plasmide pENV-3, ATCC No. 53072, l'insert env-4 du plasmide pENV-4, ATCC No. 53073, l'insert env-5 du plasmide pENV-5, ATCC No. 53074 ou en isolant une séquence d'ADN codant une protéine, laquelle protéine est homologue à au moins à 90% à la protéine codée par l'un des inserts env-1 à env-5, et où ladite séquence d'ADN est homologue à au moins à 90% avec la séquence codant env-1 à env-5 d'une manière connue.

2. Procédé de préparation d'un plasmide recombinant capable d'une réplication dans des cellules hôtes bactériennes, où des signaux procaryotes de transcription et de traduction pour l'expression en combinaison avec une séquence d'ADN obtenue selon la revendication 1 en phase de lecture sont insérés dans un plasmide.

3. Procédé selon la revendication 2 où lesdits signaux sont dérivés de l'opéron trp.

4. Méthode de production d'une cellule bactérienne où la cellule bactérienne est transformée par un plasmide recombinant obtenu selon l'une quelconque des revendications 2 ou 3.

5. Méthode selon la revendication 4, où ladite cellule bactérienne est E. coli.

6. Méthode de préparation d'une protéine qui a une activité immunologique avec les anticorps à LAV comprenant :
   l'introduction, dans une cellule hôte bactérienne, du plasmide recombinant selon la revendication 2 ou 3,
   la croissance dudit hôte bactérien dans un milieu approprié, et
   l'isolement de la protéine.

7. Méthode de la revendication 6, comprenant, après isolement de ladite protéine, la purification de ladite protéine par chromatographie par perméation de gel.

8. Méthode de préparation d'une composition pharmaceutique où une protéine produite selon l'une quelconque des revendications 6 ou 7 est combinée à un support et/ou diluant physiologiquement acceptable.

9. Méthode selon la revendication 8, où un adjuvant est ajouté à la composition.

10. Méthode de préparation d'une composition pharmaceutique à utiliser pour stimuler le système immunitaire d'un individu contre une contamination provoquée par le virus responsable du syndrome immunodéficitaire acquis où on formule une protéine produite selon l'une quelconque des revendications 6 ou 7.

11. Méthode de préparation d'une composition pharmaceutique à utiliser pour stimulater le système immunitaire d'un individu contre une contamination provoquée par le virus responsable du syndrome immunodéficitaire acquis où une protéine codée par la séquence d'ADN obtenue selon la revendication 1 est formulée.

12. Méthode de préparation d'une protéine qui comprend une portion de la protéine de l'enveloppe de LAV, laquelle protéine a une activité immunologique avec les anticorps à LAV, en produisant une protéine ayant la séquence d'acides aminés de la figure 7, à partir de l'isoleucine, No. 1 jusqu'à la thréonine, No. 173, d'une manière connue.

13. Utilisation d'une protéine selon l'une quelconque des revendications 6 ou 7, dans un essai pour déterminer la présence d'anticorps à LAV dans un fluide biologique.

14. Utilisation d'une protéine produite selon l'une quelconque des revendications 6 ou 7, dans un essai pour déterminer la présence de l'antigène de LAV dans un fluide biologique.

15. Utilisation d'une protéine produite selon l'une quelconque des revendications 6 ou 7, pour produire des anticorps à LAV.

16. Utilisation d'une protéine selon l'une quelconque des revendications 6 ou 7, pour la préparation d'un médicament.

17. Utilisation d'une protéine telle que codée par la séquence d'ADN produite selon la revendication 1, pour la préparation d'un médicament pour stimuler le système immunitaire d'un individu contre une contamination provoquée par le virus responsable du syndrome immunodéficitaire acquis.

FIG. 1

pJH11 :    5′ GAG ATC CCC GGG CGA GCT CGA ATT CGA GCT

                     ┌─BamHI─┐
                CGC CCG GGG ATC CTC TAG AGT CGA CCT GCA

               ┌─HindⅢ─┐
                GCC CAA GCT T  3′

                              ┌─BamHI─┐
pJH12    5′ GAG ATC CCC GGG GAT CCT CTA GAG TCG ACC

                  ┌─HindⅢ─┐
                TGC AGC CCA AGC TT  3′

                                   ┌─BamHI─┐
pJH14 :    5′ GAG ATC CCC CCG AAT TCG GGG GGA TCC TCT

                            ┌─HindⅢ─┐
                AGA GTC GAC CTG CAG CCC AAG CTT 3′

# FIG. 2

FIG.3

FIG. 4

FIG. 5

EP 0 201 716 B1

FIG. 6

ENV-3

# FIG. 7

<u>    &#35;5    </u>

Ile Phe Arg Pro Gly Gly Gly Asp Met Arg Asp Asn Trp Arg Ser Glu

             30

Leu Try Lys Tyr Lys Val Val Lys Ile Glu Pro Leu Gly Val Ala Pro

<u>    &#35;2  40    </u>

Thr Lys Ala Lys Arg Arg Val Val Gln Arg Glu Lys Arg Ala Val Gly

<u> 50  &#35;1   </u>  60

Ile Gly Ala Leu Phe Leu Gly Phe Leu Gly Ala Ala Gly Ser Thr Met

    70        80

Gly Ala Gly Ser Met Thr Leu Thr Val Gln Ala Arg Gln Leu Leu Ser

      90

Gly Ile Val Gln Gln Gln Asn Asn Leu Leu Arg Ala Ile Glu Ala Gln

  100       110

Gln His Leu Leu Gln Leu Thr Val Trp Gly Ile Lys Gln Leu Gln Ala

    120

Arg Ile Leu Ala Val Glu Arg Tyr Leu Lys Asp Gln Gln Leu Leu Gly

 130 <u>  &#35;3  </u> 140

Ile Trp Gly Cys Ser Gly Lys Leu Ile Cys Thr Thr Ala Val Pro Trp

    150      160

Asn Ala Ser Trp Ser Asn Lys Ser Leu Glu Gln Ile Trp Asn Asn Met

    <u>170 &#35;4  </u>

Thr Trp Met Glu Trp Asp Arg Glu Ile Asn Asn Tyr Thr

# FIG.8

```
Lys Glu Gln Lys Thr Val Ala Met Arg Val Lys Glu Lys Tyr Gln His
AAA GAG CAG AAG ACA GTG GCA ATG AGA GTG AAG GAG AAA TAT CAG CAC


Leu Trp Arg Trp Gly Trp Lys Trp Gly Thr Met Leu Leu Gly Ile Leu
TTG TGG AGA TGG GGG TGG AAA TGG GGC ACC ATG CTC CTT GGG ATA TTG
        5800


Met Ile Cys Ser Ala Thr Glu Lys Leu Trp Val Thr Val Tyr Tyr Gly
ATG ATC TGT AGT GCT ACA GAA AAA TTG TGG GTC ACA GTC TAT TAT GGG


Val Pro Val Trp Lys Glu Ala Thr Thr Thr Leu Phe Cys Ala Ser Asp
GTA CCT GTG TGG AAG GAA GCA ACC ACC ACT CTA TTT TGT GCA TCA GAT
            5900


Ala Lys Ala Tyr Asp Thr Glu Val His Asn Val Trp Ala Thr His Ala
GCT AAA GCA TAT GAT ACA GAG GTA CAT AAT GTT TGG GCC ACA CAT GCC


Cys Val Pro Thr Asp Pro Asn Pro Gln Glu Val Val Leu Val Asn Val
TGT GTA CCC ACA GAC CCC AAC CCA CAA GAA GTA GTA TTG GTA AAT GTG
                6000


Thr Glu Asn Phe Asn Met Trp Lys Asn Asp Met Val Glu Gln Met His
ACA GAA AAT TTT AAC ATG TGG AAA AAT GAC ATG GTA GAA CAG ATG CAT


Glu Asp Ile Ile Ser Leu Trp Asp Gln Ser Leu Lys Pro Cys Val Lys
GAG GAT ATA ATC AGT TTA TGG GAT CAA AGC CTA AAG CCA TGT GTA AAA
                6100


Leu Thr Pro Leu Cys Val Ser Leu Lys Cys Thr Asp Leu Gly Asn Ala
TTA ACC CCA CTC TGT GTT AGT TTA AAG TGC ACT GAT TTG GGG AAT GCT
```

```
Thr Asn Thr Asn Ser Ser Asn Thr Asn Ser Ser Ser Gly Glu Met Met
ACT AAT ACC AAT AGT AGT AAT ACC AAT AGT AGT AGC GGG GAA ATG ATG
                                6200

Met Glu Lys Gly Glu Ile Lys Asn Cys Ser Phe Asn Ile Ser Thr Ser
ATG GAG AAA GGA GAG ATA AAA AAC TGC TCT TTC AAT ATC AGC ACA AGC

Ile Arg Gly Lys Val Gln Lys Glu Tyr Ala Phe Phe Tyr Lys Leu Asp
ATA AGA GGT AAG GTG CAG AAA GAA TAT GCA TTT TTT TAT AAA CTT GAT

                        6300
Ile Ile Pro Ile Asp Asn Asp Thr Thr Ser Tyr Thr Leu Thr Ser Cys
ATA ATA CCA ATA GAT AAT GAT ACT ACC AGC TAT ACG TTG ACA AGT TGT

Asn Thr Ser Val Ile Thr Gln Ala Cys Pro Lys Val Ser Phe Glu Pro
AAC ACC TCA GTC ATT ACA CAG GCC TGT CCA AAG GTA TCC TTT GAG CCA
                                6400

Ile Pro Ile His Tyr Cys Ala Pro Ala Gly Phe Ala Ile Leu Lys Cys
ATT CCC ATA CAT TAT TGT GCC CCG GCT GGT TTT GCG ATT CTA AAA TGT

Asn Asn Lys Thr Phe Asn Gly Thr Gly Pro Cys Thr Asn Val Ser Thr
AAT AAT AAG ACG TTC AAT GGA ACA GGA CCA TGT ACA AAT GTC AGC ACA
                                6500

Val Gln Cys Thr His Gly Ile Arg Pro Val Val Ser Thr Gln Leu Leu
GTA CAA TGT ACA CAT GGA ATT AGG CCA GTA GTA TCA ACT CAA CTG CTG

Leu Asn Gly Ser Leu Ala Glu Glu Glu Val Val Ile Arg Ser Ala Asn
TTG AAT GGC AGT CTA GCA GAA GAA GAG GTA GTA ATT AGA TCT GCC AAT
                                6600

Phe Thr Asp Asn Ala Lys Thr Ile Ile Val Gln Leu Asn Gln Ser Val
TTC ACA GAC AAT GCT AAA ACC ATA ATA GTA CAG CTG AAC CAA TCT GTA
```

```
Glu Ile Asn Cys Thr Arg Pro Asn Asn Asn Thr Arg Lys Ser Ile Arg
GAA ATT AAT TGT ACA AGA CCC AAC AAC AAT ACA AGA AAA AGT ATC CGT
                                                          6700


Ile Gln Arg Gly Pro Gly Arg Ala Phe Val Thr Ile Gly Lys Ile Gly
ATC CAG AGG GGA CCA GGG AGA GCA TTT GTT ACA ATA GGA AAA ATA GGA


Asn Met Arg Gln Ala His Cys Asn Ile Ser Arg Ala Lys Trp Asn Ala
AAT ATG AGA CAA GCA CAT TGT AAC ATT AGT AGA GCA AAA TGG AAT GCC
                                                            6800


Thr Leu Lys Gln Ile Ala Ser Lys Leu Arg Glu Gln Phe Gly Asn Asn
ACT TTA AAA CAG ATA GCT AGC AAA TTA AGA GAA CAA TTT GGA AAT AAT


Lys Thr Ile Ile Phe Lys Gln Ser Ser Gly Gly Asp Pro Glu Ile Val
AAA ACA ATA ATC TTT AAG CAA TCC TCA GGA GGG GAC CCA GAA ATT GTA


Thr His Ser Phe Asn Cys Gly Gly Glu Phe Phe Tyr Cys Asn Ser Thr
ACG CAC AGT TTT AAT TGT GGA GGG GAA TTT TTC TAC TGT AAT TCA ACA
6900


Gln Leu Phe Asn Ser Thr Trp Phe Asn Ser Thr Trp Ser Thr Glu Gly
CAA CTG TTT AAT AGT ACT TGG TTT AAT AGT ACT TGG AGT ACT GAA GGG


Ser Asn Asn Thr Glu Gly Ser Asp Thr Ile Thr Leu Pro Cys Arg Ile
TCA AAT AAC ACT GAA GGA AGT GAC ACA ATC ACA CTC CCA TGC AGA ATA
        7000


Lys Gln Phe Ile Asn Met Trp Gln Glu Val Gly Lys Ala Met Tyr Ala
AAA CAA TTT ATA AAC ATG TGG CAG GAA GTA GGA AAA GCA ATG TAT GCC


Pro Pro Ile Ser Gly Gln Ile Arg Cys Ser Ser Asn Ile Thr Gly Leu
CCT CCC ATC AGC GGA CAA ATT AGA TGT TCA TCA AAT ATT ACA GGG CTG
            7100
```

```
Leu Leu Thr Arg Asp Gly Gly Asn Asn Asn Asn Gly Ser Glu Ile Phe
CTA TTA ACA AGA GAT GGT GGT AAT AAC AAC AAT GGG TCC GAG ATC TTC


Arg Pro Gly Gly Gly Asp Met Arg Asp Asn Trp Arg Ser Glu Leu Tyr
AGA CCT GGA GGA GGA GAT ATG AGG GAC AAT TGG AGA AGT GAA TTA TAT
                7200


Lys Tyr Lys Val Val Lys Ile Glu Pro Leu Gly Val Ala Pro Thr Lys
AAA TAT AAA GTA GTA AAA ATT GAA CCA TTA GGA GTA GCA CCC ACC AAG


Ala Lys Arg Arg Val Val Gln Arg Glu Lys Arg Ala Val Gly Ile Gly
GCA AAG AGA AGA GTG GTG CAG AGA GAA AAA AGA GCA GTG GGA ATA GGA
                7300


Ala Leu Phe Leu Gly Phe Leu Gly Ala Ala Gly Ser Thr Met Gly Ala
GCT TTG TTC CTT GGG TTC TTG GGA GCA GCA GGA AGC ACT ATG GGC GCA


Arg Ser Met Thr Leu Thr Val Gln Ala Arg Gln Leu Leu Ser Gly Ile
CGG TCA ATG ACG CTG ACG GTA CAG GCC AGA CAA TTA TTG TCT GGG ATA
                7400


Val Gln Gln Gln Asn Asn Leu Leu Arg Ala Ile Glu Ala Gln Gln His
GTG CAG CAG CAG AAC AAT TTG CTG AGG GCT ATT GAG GCG CAA CAG CAT


Leu Leu Gln Leu Thr Val Trp Gly Ile Lys Gln Leu Gln Ala Arg Ile
CTG TTG CAA CTC ACA GTC TGG GGC ATC AAG CAG CTG CAG GCA AGA ATC
                7500


Leu Ala Val Glu Arg Tyr Leu Lys Asp Gln Gln Leu Leu Gly Ile Trp
CTG GCT GTG GAA AGA TAC CTA AAG GAT CAA CAG CTC CTG GGG ATT TGG


Gly Cys Ser Gly Lys Leu Ile Cys Thr Thr Ala Val Pro Trp Asn Ala
GGT TGC TCT GGA AAA CTC ATT TGC ACC ACT GCT GTG CCT TGG AAT GCT
                7600
```

```
Ser Trp Ser Asn Lys Ser Leu Glu Gln Ile Trp Asn Asn Met Thr Trp
AGT TGG AGT AAT AAA TCT CTG GAA CAG ATT TGG AAT AAC ATG ACC TGG


Met Glu Trp Asp Arg Glu Ile Asn Asn Tyr Thr Ser Leu Ile His Ser
ATG GAG TGG GAC AGA GAA ATT AAC AAT TAC ACA AGC TTA ATA CAT TCC
                                              7700


Leu Ile Glu Glu Ser Gln Asn Gln Gln Glu Lys Asn Glu Gln Glu Leu
TTA ATT GAA GAA TCG CAA AAC CAG CAA GAA AAG AAT GAA CAA GAA TTA


Leu Glu Leu Asp Lys Trp Ala Ser Leu Trp Asn Trp Phe Asn Ile Thr
TTG GAA TTA GAT AAA TGG GCA AGT TTG TGG AAT TGG TTT AAC ATA ACA
                                          7800


Asn Trp Leu Trp Tyr Ile Lys Ile Phe Ile Met Ile Val Gly Gly Leu
AAT TGG CTG TGG TAT ATA AAA ATA TTC ATA ATG ATA GTA GGA GGC TTG


Val Gly Leu Arg Ile Val Phe Ala Val Leu Ser Ile Val Asn Arg Val
GTA GGT TTA AGA ATA GTT TTT GCT GTA CTT TCT ATA GTG AAT AGA GTT
                                                      7900


Arg Gln Gly Tyr Ser Pro Leu Ser Phe Gln Thr His Leu Pro Thr Pro
AGG CAG GGA TAT TCA CCA TTA TCG TTT CAG ACC CAC CTC CCA ACC CCG


Arg Gly Pro Asp Arg Pro Glu Gly Ile Glu Glu Glu Gly Gly Glu Arg
AGG GGA CCC GAC AGG CCC GAA GGA ATA GAA GAA GAA GGT GGA GAG AGA
                                                          8000


Asp Arg Asp Arg Ser Ile Arg Leu Val Asn Gly Ser Leu Ala Leu Ile
GAG AGA GAG AGA TCC ATT CGA TTA GTG AAC GGA TCC TTA GCA CTT ATC


Trp Asp Asp Leu Arg Ser Leu Cys Leu Phe Ser Tyr His Arg Leu Arg
TGG GAC GAT CTG CGG AGC CTG TGC CTC TTC AGC TAC CAC CGC TTG AGA


Asp Leu Leu Leu Ile Val Thr Arg Ile Val Glu Leu Leu Gly Arg Arg
GAC TTA CTC TTG ATT GTA ACG AGG ATT GTG GAA CTT CTG GGA CGC AGG
8100
```

```
Gly Trp Glu Ala Leu Lys Tyr Trp Trp Asn Leu Leu Gln Tyr Trp Ser
GGG TGG GAA GCC CTC AAA TAT TGG TGG AAT CTC CTA CAG TAT TGG AGT


Gln Glu Leu Lys Asn Ser Ala Val Ser Leu Leu Asn Ala Thr Ala Ile
CAG GAA CTA AAG AAT AGT GCT GTT AGC TTG CTC AAT GCC ACA GGC ATA
    8200


Ala Val Ala Glu Gly Thr Asp Arg Val Ile Glu Val Val Gln Gly Ala
GCA GTA GCT GAG GGG ACA GAT AGG GTT ATA GAA GTA GTA CAA GGA GCT


Cys Arg Ala Ile Arg His Ile Pro Arg Arg Ile Arg Gln Gly Leu Glu
TGT AGA GCT ATT CGC CAC ATA CCT AGA AGA ATA AGA CAG GGC TTG GAA
        8300


Arg Ile Leu Leu
AGG ATT TTG CTA TAA
```